# FASCICULE DE BREVET EUROPEEN

(11) **EP 3 082 754 B1**
(45) Date de publication et mention de la délivrance du brevet: **19.09.2018**
(21) Numéro de dépôt: 14821789.6
(22) Date de dépôt: 03.12.2014
(51) Int. Cl.: A61K 8/60, A61Q 13/00

(54) **UTILISATION D'ALKYLPOLYGLYCOSIDES COMME SOLUBILISANTS DE PARFUMS ET COMPOSITION PARFUMANTE LES COMPRENANT**
VERWENDUNG VON ALKYLPOLYGLUCOSIDEN ZUR LÖSUNG VON PARFÜMEN UND PARFÜMZUSAMMENSETZUNGEN DIESE ENTHALTEND
USE OF ALKYLPOLYGLUCOSIDES FOR SOLUBILISING PERFUMES AND PERFUME COMPOSITIONS COMPRISING THESE

(30) Priorité: 18.12.2013 FR 1362862
(43) Date de publication de la demande: 26.10.2016
(73) Titulaire: Société d'Exploitation de Produits pour les Industries Chimiques SEPPIC, 75321 Paris cedex 07 (FR)
(72) Inventeur: BRETON, Marie-Laure, F-81100 Castres (FR); KERVERDO, Sébastien, F-94300 Vincennes (FR); GARCEL, Stéphanie, F-81100 Castres (FR); GUILBOT, Jerôme, F-81100 Castres (FR); ROLLAND, Hervé, F-81100 Castres (FR)
(74) Mandataire: Laigneau, Amandine
(86) Numéro de dépôt international: PCT/FR2014/053150
(87) Numéro de publication internationale: WO 2015/092195

(56) Documents cités:
- EP-A1- 1 211 258
- WO-A1-96/33255
- WO-A1-2012/069730
- PHILIPPE LAPEYRE: "A sustainable solubiliser and co-surfactant for cosmetics", FOCUS ON SURFACTANTS, ELSEVIER ENGINEERING INFORMATION, INC, US , vol. 31, no. 7 1 octobre 2011 (2011-10-01), pages 26-27, XP002722546, ISSN: 1351-4210, DOI: 10.1016/S1351-4210(11)70284-0 Extrait de l'Internet: URL:http://www.wheatoleo.com/sites/default /files/july_2011_speciality_chemicals_maga zine.pdf [extrait le 2011-07-01]

## Description

La présente invention concerne les alkyl polyglycosides et leur utilisation comme solubilisants en milieu aqueux.

Les industries de la parfumerie et de la cosmétique ont un besoin récurant de développer des compositions parfumantes à concentrations réduites en alcools volatils, voire des compositions parfumantes exemptes d'alcools volatils. En effet, ces compositions parfumantes présentent de nombreux inconvénients liés à la présence de ces alcools volatils, et plus particulièrement à la présence d'éthanol, car elles provoquent un dessèchement de la peau et peuvent irriter l'épiderme, elles sont sensibles à la lumière du soleil, et elles sont difficiles à mettre sur le plan olfactif, car l'odeur de l'alcool volatil peut interférer avec les substances parfumantes.

L'eau est un substitut de choix des alcools volatils dans les compositions parfumantes, car son utilisation permet de diminuer la teneur en composés volatils organiques dans l'atmosphère, de réduire ou d'éliminer le caractère inflammable des compositions parfumantes. Par ailleurs, les industries de la parfumerie et de la cosmétique commercialisent également des compositions nécessitant la présence d'eau, comme les eaux de toilettes, les eaux de parfum, des lotions aqueuses après-rasage, des eaux de soin et des eaux fraîches. Ces produits cosmétiques parfumés aqueux sont appréciés des consommateurs car ils se caractérisent par une sensation de fraîcheur accrue.

Cependant, les substances parfumantes étant de façon générale des substances hydrophobes, il s'avère nécessaire d'utiliser au moins un tiers ingrédient permettant de les solubiliser dans la phase aqueuse des compositions parfumantes. Les tensioactifs sont généralement des agents solubilisants qui sont associés aux substances parfumantes hydrophobes pour préparer des compositions parfumantes aqueuses.

Parmi les tensioactifs couramment utilisés, il y a les tensioactifs non ioniques alcoxylés comme les polysorbates, comme les esters de lauroyl sorbitan éthoxylés à 20 moles d'oxyde d'éthylène , les esters de palmitoyl sorbitan éthoxylés à 20 moles d'oxyde d'éthylène , les esters de stéaroyl sorbitan éthoxylés à 20 moles d'oxyde d'éthylène , les esters d'oléyl sorbitan éthoxylés à 20 moles d'oxyde d'éthylène ; les alcools gras éthoxylés, les acides gras éthoxylés, les esters gras éthoxylés, et plus particulièrement les triglycérides gras éthoxylés comme l'huile de ricin hydrogénée éthoxylée à 20, 40 ou 60 moles d'oxyde d'éthylène, et plus particulièrement l'huile de ricin hydrogénée éthoxylée à 40 moles d'oxyde d'éthylène couramment commercialisée sous l'appellation « PEG-40 hydrogenated castor oil ». Ces tensioactifs éthoxylés présentent cependant l'inconvénient d'être préparés par la mise en oeuvre d'oxyde d'éthylène, dont les teneurs résiduelles, et les sous produits qu'ils génèrent, sont proscrits dans les produits cosmétiques à destination des consommateurs, impliquant alors la mise en oeuvre de procédé de purification restrictifs. De plus, les composés polyéthoxylés sont suspectés de passer facilement la barrière cutanée et de permettre ainsi un accès plus rapide de l'oxyde d'éthylène résiduel et de ses sous-produits dans l'organisme humain.

Pour ces raisons, des alternatives aux solubilisants alcoxylés ont été l'objet de nombreuses recherches, qui impliquent des structures dont les matières premières sont issues de ressources renouvelables. Parmi les structures alternatives aux solubilisants alcoxylés, on peut retenir les dérivés N-acylés d'acides aminés, les esters de polyglycérol, les alkylpolyglycosides ou des mélanges d'au moins deux de ces composés.

La demande internationale publiée sous le numéro WO 2010/106423 A2 divulgue que la combinaison d'esters de polyglycérols d'acides gras et de dérivés N-acyl glutamates permet d'atteindre un système solubilisant efficace pour solubiliser dans l'eau une large gamme d'ingrédients lipophiles, comme des huiles essentielles et des parfums.

Parmi la famille des alkyl polyglycosides, le caprylyl/caprylic glucoside est un composé couramment utilisé pour solubiliser des substances et/ou des compositions chimiques lipophiles dans l'eau, et notamment pour solubiliser des substances parfumantes lipophiles dans l'eau. Cependant, le caprylyl/ caprylic glucoside présente l'inconvénient de générer la formation de mousse en solution, et il montre des capacités limitées pour procurer de la transparence aux compositions parfumantes aqueuses le comprenant.

L'amélioration des capacités solubilisantes des alkylpolyglycosides peut être procurée par sa combinaison avec un autre agent tensioactif, comme un ester de glycérol et/ou un dérivé N-acylé d'acide aminé, et plus particulièrement un dérivé N-acylé d'un sel de l'acide glutamique.

La demande internationale publiée sous le numéro WO 2010/106423 A1 divulgue des compositions solubilisantes contenant la combinaison d'au moins un ester de polyglycérol d'acide gras comportant 8 ou 10 atomes de carbone et d'au moins un N-acyl glutamate, salifié avec un métal alcalin ou avec un alcalino-terreux ou avec un ion ammonium ou avec une amine, et dont le radical acyl comporte de 8 à 14 atomes de carbone.

La demande internationale publiée sous le numéro WO 2010/106423 A1 divulgue également l'utilisation de telles compositions pour solubiliser des huiles ou des parfums dans l'eau. L'inconvénient de cette solution réside dans le fait que la composition solubilisante n'est opérante qu'à des valeurs de pH supérieures à 7,0 en raison de la nécessiter de solubiliser entièrement l'acide glutamique dans le dérivé N-acyl glutamate.

La demande internationale publiée sous le numéro WO 96/14374 A1 divulgue un système tensioactif constitué par au moins un alkylpolyglycoside dont la chaîne alkyle comporte de 1 à 8 atomes de carbone, et par au moins un acide carboxylique de N-alkylpolyhydroxylalkylamide, ainsi que l'utilisation de ce système tensioactif comme agent solubilisant dans la préparation de compositions cosmétiques, pharmaceutiques et détergentes. La demande internationale de brevet publiée sous le numéro WO 96/14374 A1 divulgue également que les alkylpolyglycosides, constitutifs du système tensioactif solubilisant avec un acide carboxylique de N-alkylpolyhydroxylalkylamide, sont de préférence le méthyloligogly-coside, le butyloligoglycoside, l'éthylèneglycololigoglucoside et/ou le glyceryloligoglucoside.

La demande de brevet européen publiée sous le numéro EP 2 366 376 A1 divulgue une composition comportant au moins un alkylpolyglycoside dont la chaine alkyle comporte de 4 à 12 atomes de carbone, au moins un ester de polyglycérol d'un acide gras dont la chaine alcoyle comporte de 8 à 20 atomes de carbone, au moins un dérivé N-acylé d'un acide aminé dont la chaine acyle comporte de 6 à 12 atomes de carbone, et au moins un agent mouillant, dont le poids moléculaire est compris entre 100 g.mol-1 et 250 g.mol-1, sélectionné parmi le groupe constitué par les monoesters de glycérol et d'acides gras, dont la chaine alcoyle comporte de 6 à10 atomes de carbone, et les 1,2-alcanediols dont la chaine hydrocarbonée comporte de 6 à 10 atomes de carbone.

Philippe Lapeyre dans « A sustainable solubiliser and cosurfactant for cosmetics », Focus on Surfactants, Elsevier Engineering information Inc, US, vol. 31, n°7 1er octobre 2011, pages 26-27 et la demande brevet européen publiée le 05 juin 2002 sous le numéro EP 1 211 258 A1, divulguent la solubilisation d'huiles essentielles en de fragrances avec des alkyl poplyglycosides (C8-C10 et C5 alkyle polypentosides (xylose) ; Radia Easysurf 6881 et 6505).

Ces différentes solutions se caractérisent par une optimisation fine des proportions des différents constituants des compositions solubilisantes, plaçant le formulateur de compositions parfumantes aqueuses devant des difficultés d'extrapolation d'échelle de mélanges complexes et également devant une multiplication des références commerciales pour constituer le système solubilisant, générant ainsi des frais de fonctionnement supplémentaires.

L'homme du métier recherche alors une solution ne mettant en oeuvre qu'une seule substance ou une seule composition solubilisante.

La demande de brevet français publiée sous le numéro FR 2 816 517 A1 divulgue la préparation d'alkylpolyglycosides à partir d'alcools de fusel et d'au moins un sucre réducteur en milieu catalytique acide, et l'utilisation dudit alkylpolyglycoside sur alcool de fusel comme agent solubilisant d'ingrédients huileux ou liposoluble. Les alcools de fusel sont des mélanges d'alcools comprenant notamment des alcools à courtes chaînes comme l'éthanol, le propan-1-ol, le propan-2-ol, le méthyl-propan-1-ol, le butan-1-ol, le 2-méthyl butan-1-ol et le 3-méthyl butan-1-ol, qui nécessitent la mise en oeuvre de mesures de sécurité anti-déflagrantes lors de leur mise en oeuvre industrielle. La demande de brevet français publiée sous le numéro FR 2 816 517 A1 divulgue également que les alkyl polyglycosides, dont la chaine alkyle provient de l'alcool de fusel, permet « d'augmenter la solubilité de molécules lipophile dans l'eau » comme les huiles essentielles et les parfums. Cependant, les alkylpolyglycosides préparés à partir d'alcools de fusel se caractérisent par une odeur les rendant peu compatibles pour la formulation de compositions comprenant des substances parfumantes.

La demande internationale publiée sous le numéro WO 96/33255 A1 décrit des compositions anti-mousses comprenant un alkylpolyglucoside particulier, dont la chaîne alkyle est constituée par le radical 2-éthylhexyle et des agents tensioactifs non-ioniques démoussants choisis parmi ceux comprenant un ou plusieurs groupes choisis parmi les groupes mono-éthoxylés ou poly-éthoxylés, les groupes mono-propoxylés ou poly-propoxylés. Il y est enseigné que les alkylpolyglucosides à chaîne 2-éthyl hexyle sont plus efficaces que les alkyl polygylycosides à chaine n-hexyle pour solubiliser des tensioactifs non-ioniques démoussants.

La demande internationale publiée sous le numéro WO 99/21948 A1 divulgue des compositions limpides et stables à hautes concentrations alcalines, dont les propriétés moussantes sont contrôlées, contenant une grande quantité de tensioactifs non-ioniques à base d'oxyde d'alkylène et un hexylglycoside comme agent hydrotrope. Il y est enseigné que les hexylglycosides et plus particulièrement le n-hexyl polyglucoside permettent de solubiliser dans des milieux fortement alcalins des tensioactifs non-ioniques.

La demande internationale publiée sous le numéro WO 2012/069730 A1 divulgue des compositions limpides et stables à des concentrations élevées à la fois en espèces alcalines et en espèces électrolytiques, comprenant une grande quantité de tensioactifs non-ioniques à base d'oxyde d'alkylène et le n-heptyl polyglucoside comme agent hydrotrope.

Rien dans l'état de la technique n'enseigne que les alkyl polyglycosides préparés à partir de n-heptanol et connus pour leurs propriétés hydrotropes, à savoir solubiliser dans l'eau des tensioactifs peu solubles dans des milieux fortement alcalins, ou acides, ou électrolytiques, puissent constituer des agents solubilisants de substances parfumantes hydrophobes dans l'eau, n'induisant pas de formation de mousse, utilisable sur une gamme de pH comprise entre 4 et 10, et ne dénaturant pas l'odeur procurée par les substances parfumantes solubilisées.

C'est pourquoi selon un premier aspect, l'invention a pour objet l'utilisation d'une composition représentée par la formule (I) :

R-O-(G)ₚ-H (I),

dans laquelle R représente le radical n-heptyle, G représente le reste du glucose et p représente un nombre décimal supérieur ou égal à 1,05 et inférieur ou égal à 2,5, ladite composition de formule (I) consistant en un mélange de composés représentés par les formules (I₁), (I₂), (I₃), (I₄) et (I₅) :

R-O-(G)₁-H (I₁)

R-O-(G)₂-H (I₂)

R-O-(G)₃-H (I₃)

R-O-(G)₄-H (I₄)

R-O-(G)₅-H (I₅)

dans les proportions molaires respectives a₁, a₂, a₃, a₄ et a₅, telles que chacune des proportions a₁, a₂, a₃, a₄ et a₅ est supérieure ou égale à zéro et inférieure ou égale à un et que la somme a₁ + a₂ + a₃ + a₄ + a₅ est égale à un, comme agent solubilisant en solution aqueuse, d'une substance parfumante hydrophobe (A) ou d'un mélange de substances parfumantes hydrophobes (A), caractérisée en ce que ladite substance parfumante hydrophobe (A) est sélectionnée dans le groupe constitué des huiles essentielles riches en composés portant une fonction phénolique, lesdits composés portant une fonction phénolique étant sélectionnés dans le groupe constitué du thymol, du carvacol de l'eugénol, de l'iso-eugénol et de l'estragol, et des huiles essentielles riches en composés portant une fonction alcool lesdits composés portant une fonction alcool étant sélectionnés dans le groupe constitué du linalol, du géraniol, du menthol, du néomenthol, du cis-hydrate de sabinène, du citronellol, de l'a-terpinéol, du viridiflorol, du trans-nérolidol, de l'a-cadinol, du τ-cadinol, du trans-farnesol, du cis-farnesol, de l'a-bisabolol, du patchoulol et du pogostol.

La structure oligomérique (G)ₚ, peut se présenter sous toutes formes d'isoméries, qu'il s'agisse d'isomérie optique, d'isomérie géométrique ou d'isomérie de position ; elle peut aussi représenter un mélange d'isomères.

Dans la formule (I) telle que définie ci-dessus, le groupe R-O est lié à G par le carbone anomérique du reste saccharide, de manière à former une fonction acétal.
Par substance parfumante hydrophobe (A) on désigne, dans l'utilisation telle que définie ci-dessus, toute parfum hydrophobe susceptible de dégager une odeur plus ou moins persistante, ledit parfum appartenant à l'une des familles olfactives telles qu'établies par la société française des parfumeurs. Cette classification est connue de l'homme du métier et est par exemple publiée sur le site internet de cette société à l'adresse suivante : http://www.parfumeurs-createurs.org/gene/main.php?base=525

Cette classification consiste en la liste des familles d'odeur suivantes : famille hespéridée, famille florale, famille fougère, famille chypre, famille boisée, famille ambrée-orientale et famille cuir.

L'association de différentes substances parfumantes hydrophobes permet de produire une signature olfactive agréable, plus ou moins persistante.

La substance parfumante hydrophobe (A) peut être choisie parmi des substances parfumantes hydrophobes d'origine naturelle, animale, ou végétale, ou d'origine synthétique.

Parmi les substances parfumantes hydrophobes d'origine animale, il y a le musc, le castoreum, la civette, l'ambre gris, l'absolue de cire d'abeille, l'hyraceum.

Les substances parfumantes hydrophobes d'origine végétale peuvent être extraites de différentes parties des végétaux, comme de la fleur, de la feuille, de la tige, de l'écorce, du bois, de la mousse de bois, du fruit, du bourgeon, de la graine, de la racine, d'herbes, de résines de végétaux ou de lichens.

Parmi les substances parfumantes hydrophobes d'origine végétale issues de l'extraction de fleurs, il y a celles issues de l'extraction de fleurs de roses, de fleurs de jasmin, de fleurs de tubéreuses, de fleurs de champaca, de fleurs de frangipanier (Ylang-ylang), de fleurs de lotus, de fleurs de mimosa, de fleurs d'oeillets, de fleurs d'osmanthus, de fleur d'acacia, de fleurs d'oranger, de fleurs de narcisses, de fleurs de lavandes, de fleurs de gardénias.

Parmi les substances parfumantes hydrophobes d'origine végétale issues de l'extraction de bourgeons, il y a celles issues de l'extraction de bourgeons de cassis.
Parmi les substances parfumantes hydrophobes d'origine végétale issues de l'extraction de mousses, il y a celles issues de l'extraction de mousses de chêne ou de mousses de hêtre.

Parmi les substances parfumantes hydrophobes d'origine végétale issues de l'extraction de feuilles, il y a les substances parfumantes hydrophobes issues de l'extraction de feuilles d'acacia, de feuilles de basilic, de feuilles de valériane, de feuilles de gentiane, de feuilles de violette, de feuilles de géranium, de feuilles de labdanum, de feuilles de romarin, de feuilles de patchouli, de feuilles de verveine.

Parmi les substances parfumantes hydrophobes d'origine végétale issues de l'extraction d'écorce, il y a les substances parfumantes hydrophobes issues de l'extraction d'écorce de cannelle, d'écorce de frêne, d'écorce de cassia, d'écorce de cascarille, d'écorce de bouleau.

Parmi les substances parfumantes hydrophobes d'origine végétale issues de l'extraction du bois, il y a les substances parfumantes hydrophobes issues de l'extraction du bois de santal, du bois de cèdre, du bois de palissandre, du bois d'aloès, du bois de gaïac.

Parmi les substances parfumantes hydrophobes d'origine végétale issues de l'extraction de résines, il y a les substances parfumantes hydrophobes issues de l'extraction de la résine de labdanum, de la résine d'élémi, du baume du Pérou, du baume du tolu, de la résine de Benjoin, de la résine de l'arbre à myrrhe.

Parmi les substances parfumantes hydrophobes d'origine végétale issues de l'extraction de résines, il y a l'encens, l'opoponax, du guggul.

Parmi les substances parfumantes hydrophobes d'origine végétale issues de l'extraction d'herbes ou de graminés, il y a les substances parfumantes hydrophobes issues de l'extraction de l'estragon, de la sauge, du thym, du basilic, du « lemon grass ».

Parmi les substances parfumantes hydrophobes d'origine végétale issues de l'extraction de racines, il y a les substances parfumantes hydrophobes issues de l'extraction de racines d'angélique, de céleri, de cardamone, d'iris, d'acore, de cactus, de vétiver.

Parmi les substances parfumantes hydrophobes d'origine végétale issues de l'extraction de fruits ou de graines, il y a les substances parfumantes hydrophobes issues de l'extraction de gousses de vanille, de graines de coriandre, de graines de badiane, de graines de fenouil, de baies de genièvre, de graines de cardamone, de graines de cumin, de clous de girofle, de fèves de tonka, d'amandes amères, d'agrumes comme de citron jaune, de citron vert, d'orange, de mandarine, de bergamote.

Parmi les substances parfumantes hydrophobes d'origine végétale, il y a les extraits, les absolues, les alcoolats et les huiles essentielles.

Par huile essentielle, on désigne dans la présente demande un produit odorant, répondant à la définition retenue par la norme ISO 9235, généralement de composition complexe, obtenu à partir d'une matière première végétale botaniquement définie, soit par entrainement à la vapeur d'eau, soit par distillation sèche, soit par un procédé mécanique approprié sans chauffage. L'huile essentielle est généralement séparée de la phase aqueuse par un procédé physique n'entraînant pas de changement significatif de sa composition. La matière première végétale mise en oeuvre pour l'obtention de l'huile essentielle peut être fraîche, flétrie, sèche, entière, contusée ou pulvérisée. L'huile essentielle peut en outre subir une étape de procédé ultérieure appropriée, comme une étape de procédé ultérieure permettant d'aboutir à une huile essentielle déterpénée, désesquiterpénée, rectifiée, privée d'un constituant identifié et nommé, décolorée.

Par huile essentielle déterpénée, on désigne une huile essentielle, telle que définie ci-dessus, privée partiellement ou totalement d'hydrocarbures monoterpéniques.
Par huile essentielle désesquiterpénée, on désigne une huile essentielle, telle que définie ci-dessus, privée partiellement ou totalement d'hydrocarbures monoterpéniques et sesquiterpéniques.

Par huile essentielle rectifiée, on désigne une huile essentielle, telle que définie ci-dessus, qui a subi une distillation fractionnée dans le but de supprimer certains constituants ou d'en modifier la teneur.

Les huiles essentielles telles que définies ci-dessus comprennent un mélange de molécules différentes, et lesdites huiles essentielles peuvent être classées dans différentes familles en fonction de la nature chimique de leurs composants majoritaires. Ainsi, on peut définir :
- La classe des huiles essentielles riches en carbures terpéniques et en carbures sesquiterpéniques,
- La classe des huiles essentielles riches en composés portant une fonction alcool ou monoterpinol,
- La classe des huiles essentielles riches en composés portant une fonction ester,
- La classe des huiles essentielles riches en composés portant une fonction aldéhyde,
- La classe des huiles essentielles riches en composés portant une fonction cétone,
- La classe des huiles essentielles riches en composés portant une fonction phénolique,
- La classe des huiles essentielles riches en composés portant une fonction éther,
- La classe des huiles essentielles riches en oxydes sesquiterpéniques,
- La classe des huiles essentielles riches en composés portant au moins un atome de soufre,

Par huiles essentielles riches en composés portant une fonction alcool, on désigne les huiles essentielles comprenant pour 100% de leur masse, une proportion massique supérieure ou égale à 35 % de composés portant une fonction alcool.

Parmi les composés portant une fonction alcool couramment présents dans les huiles essentielles, il y a les monoterpénols, comme le linalol, le géraniol, le menthol, le néomenthol, le cis-hydrate de sabinène, le citronnellol, l'a-terpinéol, les sesquiterpénols comme le viridiflorol, le trans-nérolidol, l'a-cadinol, le τ-cadinol, le trans-farnesol, le cis-farnésols, l'a-bisabolol, le patchoulol ou le pogostol.

Parmi les huiles essentielles riches en composés portant une fonction alcool, il y a celle de Géranium de type Bourbon, de Géranium Egypte, de lavande Aspic, de Rosalina, de coriandre, de bois de rose, de fleurs de rose, de Palmarosa, de menthe poivrée, de monarde fistuleuse, de Lavandin Super, de Lavandin Grosso, ou de Cabreuva.

Par huile essentielle riche en composés portant une fonction phénolique, on désigne les huiles essentielles comprenant pour 100% de leur masse, une proportion massique supérieure ou égale à 35 % de composés portant une fonction phénolique.

Parmi les composés portant une fonction phénolique couramment présents dans les huiles essentielles, il y a par exemple le thymol, le carvacol, l'eugénol, l'iso-eugénol ou l'estragol.

Parmi les huiles essentielles riches en composés portant une fonction phénolique, il y a celle de thym, de sarriette, d'origan, de cannelle de Ceylan, de basilic, ou de clou de girofle.

Les substances parfumantes hydrophobes d'origine synthétique sont obtenues par réaction chimique entre au moins deux composés chimiques, eux-mêmes d'origine naturelle ou synthétique.

Parmi les substances parfumantes hydrophobes d'origine synthétique il y a des alcools comme l'alcool benzylique, le 3,7-diméthyl-1-octanol, l'alcool isononylique, l'a- terpinéol, le menthol, le linalol, le citronellol, l'eucalyuptol ou 1,8-cinéole, le géraniol, le phytol, l'iso-phytol, l'a-terpinéol, le tétrahydrolinalol, le farnésol, le carotol, le nérol, le globulol, le vétivérol, le nérolidol, le dihydromyrcénol, le tétrahydromyrcénol, l'alcool fenchylique, le di-méthyle benzyle carbinol, l'alcool cinnamique, le 2-phényl éthanol ou l'undécavertol.

Parmi les substances parfumantes hydrophobes d'origine synthétique il y a des dérivés phénoliques comme l'anéthol, le safrol, l'isosafrol, l'eugénol, l'iso-eugénol, le gaïacol ou 2-méthoxy phénol, le chavicol (ou 4-allyl phénol), l'estragol (ou 1-allyl 4-méthoxy benzène), l'alcool cumique, le thymol ou le para-crésol.

Selon un aspect plus particulier, l'invention a pour objet l'utilisation telle que décrite précédemment pour laquelle, la substance parfumante hydrophobe (A) est sélectionnée dans le groupe constitué des huiles essentielles de thym, de sarriette, d'origan, de cannelle de Ceylan, de basilic, de clou de girofle, de Géranium de type Bourbon, de Géranium Egypte, de lavande Aspic, Rosalina, de coriandre, de bois de rose, de fleurs de rose, Palmarosa, de menthe poivrée, de monarde fistuleuse, de Lavandin Super ou de Lavandin Grosso.

Selon un autre aspect particulier, l'invention a pour objet l'utilisation telle que définie précédemment pour laquelle le rapport massique entre ladite substance parfumante hydrophobe (A) et la composition représentée par la formule (I) est supérieur ou égal à 1/7 et inférieur ou égal à 2/1.

L'invention a aussi pour objet une solution aqueuse (C₂) telle que définie à la revendication 3.

Selon un aspect plus particulier, l'invention a pour objet une solution aqueuse (C₂) telle que définie précédemment comprenant pour 100% de sa masse :
**a)** - de 0,7% à 56% massique, plus particulièrement de 0,7 % à 35 % massique d'au moins une composition représentée par la formule (I) telle que définie précédemment
**b)** - de 0,1 % à 8 % massique, plus particulièrement de 0,1 % à 5% massique d'au moins une substance parfumante hydrophobe (A) telle sélectionnée ci-dessus;
**c)** - de 36 % à 99,2 % massique, plus particulièrement de 60 % à 99,2 % massique d'eau.

Selon un autre aspect particulier, l'invention a pour objet une solution aqueuse (C₂) telle que définie précédemment dans laquelle le rapport massique entre ladite substance parfumante hydrophobe (A) et la composition représentée par la formule (I) est supérieur ou égal à 1/7 et inférieur ou égal à 2/1.

Dans le cadre de la présente invention, par « solution aqueuse » on désigne les compositions (C₂) telle que définie précédemment se présentant sous la forme d'une solution monophasique.

Selon un aspect particulier, l'invention a pour objet une solution aqueuse (C₂) telle que définie précédemment qui présente une turbidité supérieure ou égale à 1 NTU et inférieure ou égale à 100 NTU, plus particulièrement supérieure ou égale à 1 NTU et inférieure ou égale à 50 NTU, et encore plus particulièrement supérieure ou égale à 1 NTU et inférieure ou égale à 25 NTU, ladite turbidité étant mesurée quantitativement par une méthode optique néphélométrique, notamment par l'intermédiaire d'un turbidimètre optique de marque HF Scientific de modèle DRT100B.

La solution aqueuse (C₂) selon l'invention est préparée par mélange de ses constituants, sous agitation mécanique, à une vitesse d'agitation comprise entre 50 tours/minute et 500 tours/minutes, plus particulièrement entre 50 tours/minutes et 100 tours/minutes, à une température comprise entre 20°C et 80°C, plus particulièrement entre 20°C et 60°C, et encore plus particulièrement entre 20°C et 45°C.

La solution aqueuse (C₂) telle que définie précédemment peut en outre comprendre des ingrédients et des additifs usuellement utilisés dans le domaine des parfums et des compositions parfumantes, comme des solvants volatils, des antioxydants hydrosolubles, des agents séquestrants hydrosolubles, des colorants hydrosolubles, des agents de stabilisation de la couleur, des agents peptisants, des agents hydratants hydrosolubles, des vitamines hydrosolubles, des propulseurs.

Parmi les solvants volatils que l'on peut associer à la solution aqueuse (C₂) selon l'invention, il y a les alcools hydrosolubles et volatils, comme l'éthanol, l'isopropanol ou le butanol, et plus particulièrement l'éthanol, les solvants organiques comme le glycérol, le diglycérol, les oligomères du glycérol, l'éthylène glycol, le propylène glycol, le butylène glycol, le 1,3-propanediol, le 1,2-propanediol, l'hexylène glycol, le diéthylène glycol, le xylitol, l'érythritol, le sorbitol.

Selon un aspect particulier, la composition (C₂) selon l'invention comprend pour 100% de sa masse jusqu'à 20% massique d'un ou de plusieurs solvants volatils choisis parmi, l'éthanol, l'isopropanol ou le n-butanol.

Selon un autre aspect particulier, la composition (C₂) selon l'invention ne comprend pas de solvants volatils.

Parmi les agents antioxydants hydrosolubles que l'on peut associer à la solution aqueuse (C₂) selon l'invention, il y a l'acide ascorbique, la glutathione, l'acide tartarique, l'acide oxalique, le tétra sodium glutamate diacétate.
Parmi les agents séquestrants hydrosolubles que l'on peut associer à la solution aqueuse (C₂) selon l'invention, il y a les sels de l'acide éthylène diamine tétra acétique (EDTA), comme le sel de sodium de l'EDTA, les sels de l'acide diéthylène triamine penta acétique (DTPA) comme les sels de sodium du DTPA.

Parmi les colorants hydrosolubles que l'on peut associer à la solution aqueuse (C₂) selon l'invention, il y a le caramel, le Yellow 5, l'Acid Blue 9/ Blue 1, le Green 5, le Green 3 / Fast Green FCF 3, l'Orange 4, le Red 4 / Food Red 1, le Yellow 6, l'Acid Red 33 / Food Red 12, le Red 40, le carmine de cochenille (Cl 15850, Cl 75470), l'Ext. Violet 2, le Red 6-7, le Ferric Ferrocyanide, les Ultramarines, l'Acide Yellow 3 / Yellow 10, l'Acid Blue 3, le Yellow 10.

Parmi les agents hydrosolubles stabilisant de la couleur que l'on peut associer à la solution aqueuse (C₂) selon l'invention, il y a le citrate de Tris(tétramethyl hydroxy piperidinol), Benzotriazolyl Butylphenol Sulfonate de sodium, le Benzotriazolyl dodecyl p-Crésol.

Parmi les agents permettant d'amélioration la limpidité et/ou de diminuer les phénomènes de précipitation à froid que l'on peut associer à la solution aqueuse (C₂) selon l'invention, il y a les alkyléthers de cellulose, comme le méthyléther de cellulose, l'éthyléther de cellulose, le méthyl éthyl éther de cellulose.

Parmi les agents hydratants hydrosolubles que l'on peut associer à la solution aqueuse (C₂) selon l'invention, il y a le glycérol, les oligomère du glycérol, comme le diglycérol, le triglycérol, l'urée, les hydroxyurées, le glycérolglucoside, le diglycérolglucoside, les polyglycérylglucosides, l'érythritylglucoside, le sorbitylglucoside, le xylitylglucoside, la composition commercialisée sous le nom de marque AQUAXYL™ comprenant du xylitylglucoside, de l'anhydroxylitol et du xyllitol.

Lorsqu'elle se présente sous forme d'une lotion, la solution aqueuse (C₂) selon l'invention telle que définie précédemment peut s'appliquer comme eau fraîche, comme eau de toilette, comme eau de parfum, comme lotion d'après-rasage.

Lorsqu'elle est conditionnée dans des flacons, la solution aqueuse (C₂) selon l'invention telle que définie précédemment peut être appliquée sous la forme d e fines gouttelettes au moyen de dispositifs de pressurisation mécanique ou à gaz propulseur.

Parmi les agents propulseurs que l'on peut associer à la solution aqueuse (C₂) selon l'invention, il y a les composés hydrofluorés, comme le dichlorodifluorométhane, le trichlorofluorométhane, le difluoréthane, l'isobutane, le n-butane, le propane.

L'invention a aussi pour objet un procédé pour parfumer la peau, les cheveux, le cuir chevelu, les muqueuses ou un vêtement, comprenant une étape d'application sur ladite peau, lesdits cheveux, ledit cuir chevelu, lesdites muqueuses ou ledit vêtement, de la solution aqueuse (C₂) telle que définie précédemment.

L'invention a aussi pour objet l'utilisation de la solution aqueuse (C₂) telle que définie précédemment comme agent parfumant pour préparer une composition cosmétique (C₃) à usage topique.

Par « agent parfumant », on désigne l'action par ledit agent de conférer une odeur, plus ou moins persistante, à une composition cosmétique (C₃) à usage topique.

L'expression "à usage topique" signifie que la composition (C₃) selon l'invention est mise en oeuvre par application sur la peau, les cheveux, le cuir chevelu ou les muqueuses, qu'il s'agisse d'une application directe ou d'une application indirecte, lorsque par exemple la composition (C₃) selon l'invention est incorporée dans un support destiné à être mis en contact avec la peau (papier, lingette, textile, dispositif transdermique, etc.).

La composition cosmétique (C₃) à usage topique peut se présenter sous la forme d'un gel aqueux, d'une émulsion ou d'une microémulsion, qui peut être du type huile-dans-eau (H/E), eau-dans-huile (E/H), huile-dans-eau-dans-huile (H/E/H), eau-dans-huile-dans-eau (E/H/E). La phase huile de l'émulsion ou de la microémulsion peut consister en un mélange d'une ou de plusieurs huiles.

Lorsque la composition cosmétique (C₃) à usage topique se présente sous la forme d'un gel aqueux (GA), ledit gel aqueux (GA) comprend une quantité efficace d'au moins un agent épaississant et/ou gélifiant.

Par quantité efficace d'au moins un agent épaississant et/ou gélifiant, on désigne une quantité massique telle qu'elle permet d'augmenter la viscosité dynamique de la solution aqueuse (C₂) telle que définie précédemment à la valeur souhaitée. La quantité efficace d'un tel agent épaississant et/ou gélifiant, pour 100% de la masse de ladite composition cosmétique (C₃) à usage topique se présentant sous la forme d'un gel aqueux (GA), est généralement supérieure ou égale à 0,05% massique et inférieure ou égale à 5% massique, plus particulièrement supérieure ou égale à 0,1% massique et inférieure ou égale à 5% massique, et encore plus particulièrement supérieure ou égale à 0,1% massique et inférieure ou égale à 3% massique.

De façon générale, la viscosité dynamique de la composition cosmétique (C₃) à usage topique se présentant sous la forme d'un gel aqueux (GA), mesurée à 20°C à l'aide d'un viscosimètre de type Brookfield LV à une vitesse de 5 tours/min avec un mobile adapté, est supérieure ou égale à 5 000 mPa.s et inférieure ou égale à 200 000 mPa.s, plus particulièrement supérieure ou égale à 5 000 mPa.s et inférieure ou égale à 100 000 mPa.s, et encore plus particulièrement supérieure ou égale à 10 000 mPa.s et inférieure ou égale à 80 000 mPa.s.

Selon un aspect particulier, l'agent épaississant et/ou gélifiant compris dans la composition cosmétique (C₃) à usage topique se présentant sous la forme d'un gel aqueux (GA) est sélectionné parmi les polysaccharides constitués uniquement d'oses.

Selon un aspect plus particulier, l'agent épaississant et/ou gélifiant compris dans la composition cosmétique (C₃) à usage topique se présentant sous la forme d'un gel aqueux (GA) est sélectionné parmi les polysaccharides constitués uniquement d'oses choisis parmi les éléments du groupe constitué par les glucanes ou homopolymères du glucose, les glucomannoglucanes, les xyloglycanes, les galactomannanes dont le degré de substitution (DS) des unités de D-galactose sur la chaîne principale de D-mannose est compris entre 0 et 1, et plus particulièrement entre 0,25 et 1, comme les galactomannanes provenant de la gomme de cassia (DS = 1/5), de la gomme de caroube (DS = 1/4), de la gomme de tara (DS = 1/3), de la gomme de guar (DS = 1/2), de la gomme de fenugrec (DS = 1).

Selon un aspect encore plus particulier, l'agent épaississant et/ou gélifiant compris dans la composition cosmétique (C₃) à usage topique se présentant sous la forme d'un gel aqueux (GA) est sélectionné parmi les polysaccharides constitués uniquement d'oses choisis parmi les éléments du groupe constitué par les galactomannanes provenant de la gomme de caroube (DS = 1/4), de la gomme de tara (DS = 1/3), de la gomme de guar (DS = 1/2).

Selon un autre aspect particulier, l'agent épaississant et/ou gélifiant compris dans la composition cosmétique (C₃) à usage topique se présentant sous la forme d'un gel aqueux (GA) est sélectionné parmi les polysaccharides constitués de dérivés d'oses.

Selon un aspect plus particulier, l'agent épaississant et/ou gélifiant compris dans la composition cosmétique (C₃) à usage topique se présentant sous la forme d'un gel aqueux (GA) est sélectionné parmi les polysaccharides constitués de dérivés d'oses choisis parmi les éléments du groupe constitué par les galactanes sulfatés et plus particulièrement les carraghénanes et l'agar, les uronanes et plus particulièrement les algines, les alginates et les pectines, les hétéropolymères d'oses et d'acides uroniques et plus particulièrement la gomme xanthane, la gomme gellane, les exsudats de gomme arabique et de gomme de karaya, les glucosaminoglycanes.

Selon un aspect encore plus particulier, l'agent épaississant et/ou gélifiant compris dans la composition cosmétique (C₃) à usage topique se présentant sous la forme d'un gel aqueux (GA) est sélectionné parmi les polysaccharides constitués de dérivés d'oses choisis parmi les éléments du groupe constitué par les alginates, la gomme xanthane, les exsudats de gomme arabique.

Selon un aspect particulier, l'agent épaississant et/ou gélifiant compris dans la composition cosmétique (C₃) à usage topique se présentant sous la forme d'un gel aqueux (GA) est sélectionné parmi la cellulose, les dérivés de cellulose comme la méthyl-cellulose, l'éthyl-cellulose, l'hydroxypropyl cellulose, les silicates, l'amidon, les dérivés hydrophiles de l'amidon, les polyuréthanes.

Selon un aspect particulier, l'agent épaississant et/ou gélifiant compris dans la composition cosmétique (C₃) à usage topique se présentant sous la forme d'un gel aqueux (GA) est sélectionné parmi les polymères de type polyélectrolytes, linéaires ou branchés et/ou réticulés.

Selon un aspect plus particulier, l'agent épaississant et/ou gélifiant compris dans la composition cosmétique (C₃) à usage topique se présentant sous la forme d'un gel aqueux (GA) est sélectionné dans le groupe constitués des polymères de type polyélectrolytes, linéaires, branchés et/ou réticulés suivants :
- Les homopolymères de l'acide acrylique totalement ou partiellement salifié, de l'acide méthacrylique totalement ou partiellement salifié, de l'acide 2-méthyl [(1-oxo-2-propényl) amino] 1-propanesulfonique (AMPS) totalement ou partiellement salifié ;
- Le copolymère de l'acide acrylique ou méthacrylique totalement ou partiellement salifié et de l'acide 2-méthyl [(1-oxo-2-propényl) amino] 1-propanesulfonique (AMPS) totalement ou partiellement salifié ;
- Les copolymère de l'acide acrylique ou méthacrylique totalement ou partiellement salifié et d'un monomère neutre choisi parmi l'acrylamide, l'acrylate de (2-hydroxy éthyle), les N,N-dialkyl acrylamide comme le N,N-diméthyl acrylamide, la N-vinyl pyrrolidone, le méthacrylate de (2-hydroxy éthyle), le tris(hydroxy methyl) acrylamido méthane (THAM) et le N-(2-hydroxy éthyl) acrylamide ;
- Les copolymère de l'acide 2-méthyl [(1-oxo-2-propényl) amino] 1-propanesulfonique (AMPS) totalement ou partiellement salifié et d'un monomère neutre choisi parmi l'acrylamide, l'acrylate de (2-hydroxy éthyle), les N,N-dialkyl acrylamide comme le N,N-diméthyl acrylamide, la N-vinyl pyrrolidone, le méthacrylate de (2-hydroxy éthyle), le tris(hydroxy methyl) acrylamido méthane (THAM) et le N-(2-hydroxy éthyl) acrylamide ;
- Les terpolymères de l'acide 2-méthyl [(1-oxo-2-propényl) amino] 1-propanesulfonique (AMPS) totalement ou partiellement salifié, de l'acide acrylique ou méthacrylique totalement ou partiellement salifié, et d'un monomère neutre choisi parmi l'acrylamide, l'acrylate de (2-hydroxy éthyle), les N,N-dialkyl acrylamide comme le N,N-diméthyl acrylamide, la N-vinyl pyrrolidone, le méthacrylate de (2-hydroxy éthyle), le tris(hydroxy methyl) acrylamido méthane (THAM) et le N-(2-hydroxy éthyl) acrylamide ;
- Les terpolymères de l'acide-2-méthyl-[(1-oxo-2-propényl)amino] 1-propanesulfonique (AMPS) totalement ou partiellement salifié, de l'acide acrylique totalement ou partiellement salifié et de l'acide méthacrylique totalement ou partiellement salifié ;
- Les copolymères de l'acide acrylique ou de l'acide méthacrylique et d'acrylates d'alkyle dont la chaîne carbonée comprend entre quatre et trente atomes de carbone et plus particulièrement entre dix et trente atomes de carbone ;
- Les copolymères de l'acide 2-méthyl [(1-oxo-2-propényl) amino] 1-propanesulfonique (AMPS) totalement ou partiellement salifié et d'acrylates ou de méthacrylates d'alkyle dont la chaîne alkyle carbonée comprend entre quatre et trente atomes de carbone et plus particulièrement entre dix et trente atomes de carbone ;
- Les polymères, et plus particulièrement les copolymères, les terpolymères et les tétrapolymères réticulés comprenant de l'acide 2-méthyl [(1-oxo-2-propényl)amino] 1-propanesulfonique (AMPS) totalement ou partiellement salifié et des macromères, comme ceux décrits et revendiqués dans la demande de brevet européen publiée sous le numéro EP 1 069 142 A1, dans la demande de brevet européen publiée sous le numéro EP 1 339 789 A2, dans la demande internationale publiée sous le numéro WO 2011/030044 A1 et dans la demande de brevet de brevet français publiée sous le numéro FR 2 910 899 A1.

Lorsque la composition cosmétique (C₃) à usage topique se présente sous la forme d'une émulsion (E₁), ladite émulsion (E₁) comprend une phase grasse (P₁) constituée d'au moins une huile et/ou au moins une cire, un système émulsionnant (S₁) comprenant au moins un tensioactif émulsionnant.

Par huiles, on entend dans la présente demande de brevet, les composés et/ou les mélanges de composés insolubles dans l'eau, se présentant sous un aspect liquide à une température de 25°C. Parmi les huiles pouvant constituer la phase grasse (P₁) comprise dans la composition cosmétique (C₃) à usage topique se présente sous la forme d'une émulsion (E₁), il y a :
- Les huiles minérales telles que l'huile de paraffine, l'huile de vaseline, les isoparaffines ou les huiles blanches minérales ;
- Les huiles d'origine animale, telles que le squalène ou le squalane ;
- Les huiles végétales, telles que le phytosqualane, le beurre de karité ou les huiles d'amandes douces, de coprah, de ricin, de jojoba, d'olive, de colza, d'arachide, de tournesol, de germes de blé, de germes de maïs, de soja, de coton, de luzerne, de pavot, de potiron, d'onagre, de millet, d'orge, de seigle, de carthame, de bancoulier, de passiflore, de noisette, de palme, de noyau d'abricot, de calophyllum, de sysymbrium, d'avocat, de calendula ou encore celles issues de fleurs ou de légumes ;
- les huiles végétales éthoxylées ;
- les huiles synthétiques tels que :
- Les esters d'acides gras comme les myristates de butyle, de propyle ou de cétyle, le palmitate d'isopropyle, les stéarates de butyle, d'hexadécyle, d'isopropyle, d'octyle ou d'isocétyle, l'oléate dodécyle, le laurate d'hexyle ou le dicaprylate de propylèneglycol ;
- Les esters de l'acide lanolique, tels que les lanolates d'isopropyle, ou d'isocétyle ;
- Les monoglycérides, diglycérides et triglycérides d'acides gras comme le triheptanoate de glycérol ; ou encore
- Les alkylbenzoates, les huiles hydrogénées, les poly(α-oléfine), les polyoléfines comme le poly(isobutane), les iso-alcanes de synthèse comme l'isohexadécane, l'isododécane, les huiles perfluorées et les huiles de silicone comme les diméthylpolysiloxanes, les méthylphényl - polysiloxanes, les silicones modifiées par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles.

Par cires, on entend dans la présente demande de brevet, les composés et/ou les mélanges de composés insolubles dans l'eau, se présentant sous un aspect solide à une température supérieure ou égale à 45°C. Parmi les cires pouvant constituer la phase grasse (P₁) comprise dans la composition cosmétique (C₃) à usage topique se présente sous la forme d'une émulsion (E₁), il y a les cires d'abeille, de carnauba, de candelilla, d'ouricoury, du Japon, de fibre de liège, de canne à sucre, de paraffines, de lignite, de lanoline, polyéthylène ou de silicone ou les cires microcristallines, les cires végétales, l'ozokérite, les alcools gras et les acides gras solides à température ambiante ou les glycérides solides à température ambiante.

Parmi les autres matières grasses que l'on peut associer à la phase grasse (P₁) comprise dans la composition cosmétique (C₃) à usage topique se présentant sous la forme d'une émulsion (E₁), il y a les alcools gras saturés ou insaturés, linéaires ou ramifiés, ou les acides gras saturés ou insaturés, linéaires ou ramifiés.

Parmi les tensioactifs émulsionnants pouvant constituer le système émulsionnant (S₁) compris dans la composition cosmétique (C₃) à usage topique se présentant sous la forme d'une émulsion (E₁), il y a des tensioactifs émulsionnants choisis parmi les tensioactifs émulsionnants amphotères, anioniques, cationiques ou non ioniques ; les tensioactifs émulsionnants sont choisis en fonction du sens de l'émulsion (E₁), à savoir une émulsion (E₁₁) de type eau-dans-huile (E/H) ou une émulsion (E₁₂) de type huile-dans-eau (H/E).

Parmi les tensioactifs émulsionnants pouvant constituer le système émulsionnant (S₁) compris dans la composition cosmétique (C₃) à usage topique se présentant sous la forme d'une émulsion (E₁₁) de type eau-dans-huile, il y a :
- Les alcools gras éthoxylés, dont la chaîne grasse alkyle, saturée ou insaturée, linéaire ou ramifiée, comporte de huit à trente-six atomes de carbone et plus particulièrement de douze à vingt-deux atomes de carbone et dont le degré d'éthoxylation est compris entre 1 et 5, et plus particulièrement entre 1 et 3 ;
- Les acides gras et éthoxylés, dont la chaîne grasse alkyle, saturée ou insaturée, linéaire ou ramifiée, comporte de huit à trente-six atomes de carbone et plus particulièrement de douze à vingt-deux atomes de carbone et dont le degré d'éthoxylation est compris entre 1 et 5, et plus particulièrement entre 1 et 3 ;
- Les esters d'huiles végétales comme ceux d'huile de colza, d'huile de tournesol ou, d'huile de maïs ;
- Les esters d'huiles végétales éthoxylées comme l'ester méthylique d'huile de colza éthoxylé avec une quantité d'oxyde d'éthylène comprise entre 1 et 5 moles, plus particulièrement entre 1 et 3 mole ;
- Les esters gras de glycérol dont la chaîne grasse alkyle, saturée ou insaturée, linéaire ou ramifiée, comporte de huit à trente-six atomes de carbone et plus particulièrement de dix à vingt-deux atomes de carbone et encore plus particulièrement de douze à dix-huit atomes de carbone, comme le stéarate de glycérol, l'oléate de glycérol, le palmitate de glycérol ou l'isostérate de glycérol ;
- Les esters gras d'oligoglycérol dont la chaîne grasse alkyle, saturée ou insaturée, linéaire ou ramifiée, comporte de huit à vingt-deux atomes de carbone, et dont le nombre de moles de glycérol est compris entre 2 et 10, comme les isostéarates de tétraglycérol, de décaglycérol, ou de diglycérol, le laurate de diglycérol, les palmitates de diglycérol ou de tétraglycérol, l'oléate de décaglycérol, le monolaurate de décaglycérol, le monolinoléate de décaglycérol ou le mono-myristate de décaglycérol ;
- Les esters gras de sorbitan dont la chaîne grasse alkyle, saturée ou insaturée, linéaire ou ramifiée, comporte de huit à dix-huit atomes de carbone, et plus particulièrement de douze à dix-huit atomes de carbone, comme le monooléate de sorbitan, le dioléate de sorbitan, le trioléate de sorbitan, le tétraoléate de sorbitan, le monostéarate de sorbitan, le distéarate de sorbitan, le tristéarate de sorbitan, le tétrastéarate de sorbitan, le monoisostéarate de sorbitan, le diisostéarate de sorbitan, le triisostéarate de sorbitan, le tétraisostéarate de sorbitan, le monopalmitate de sorbitan, le dipalmitate de sorbitan, le tripalmitate de sorbitan, le tétrapalmitate de sorbitan, le monolaurate de sorbitan, le dilaurate de sorbitan, le trilaurate de sorbitan ou le tétralaurate de sorbitan ;
- Les compositions d'alkylpolyglycosides, celles d'alkylpolyglucosides, comme celles d'isostéarylpolyglucosides, d'alkylpolyxylosides, comme celles d'isostéarylxylosides ou les compositions d'octyl-2 dodécylpolyxylosides ;
- Les compositions d'alkylpolyglycosides et d'alcools gras, comme celles d'alkylpolyglucosides et d'alcools gras, par exemple les celles d'isostéarylpolyglucosides et d'alcool isostéarylique ; ou les compositions d'alkylpolyxylosides et d'alcools gras, comme celles d'isostéarylxylosides et d'alcool isostéarylique ou celles d'octyl-2 dodécylpolyxylosides et de 2-octyl dodécyle commercialisées sous le nom de marque FLUIDANOV™20X ;
- Les polyhydroxystéarates de polyglycols représentés par la formule (II) : 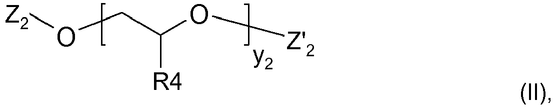 dans laquelle y₂ représente un nombre entier supérieur ou égal à 2 et inférieur ou égal à 50, R₄ représente l'atome d'hydrogène, le radical méthyle, ou le radical éthyle, Z₂ représente un radical de formule (III) : 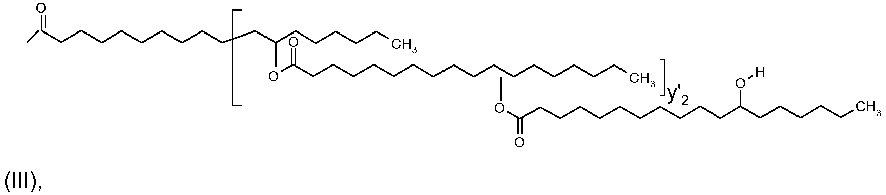 dans laquelle y'₂ représente un nombre entier supérieur ou égal à 0 et inférieur ou égal à 10, plus particulièrement supérieur ou égal à 1 et inférieur ou égal à 10 ;
   et Z'₂ représente un radical de formule (III) telle que définie ci-dessus, avec Z₂' identique ou différent de Z₂, ou l'atome d'hydrogène.
- Les polyhydroxystéarates de polyglycols représentés par la formule (IV) : 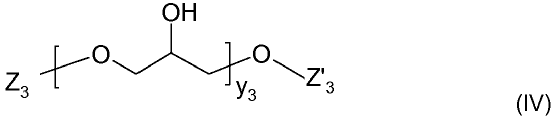 dans laquelle Z₃ représente un radical de formule (III) telle que définie précédemment, Z'₃ représente un radical de formule (III) telle que définie précédemment, avec Z₃' identique ou différent de Z₃, ou l'atome d'hydrogène et y₃ représente un nombre entier supérieur ou égal à 2 et inférieur ou égal à 20.
- Les polyhydroxystéarates de polyglycérols alcoxylés représentés par la formule (V): 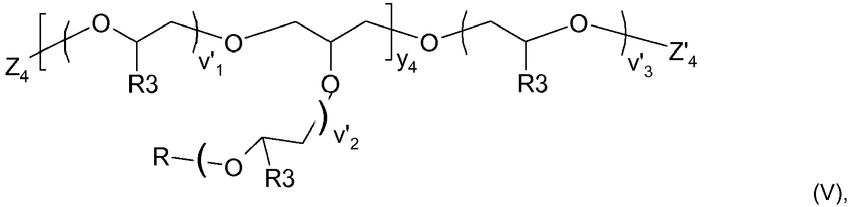 dans laquelle Z₄ représente un radical de formule (III) telle que définie ci-dessus, Z'₄ représente un radical de formule (III) telle que définie ci-dessus, avec Z₄' identique ou différent de Z₄, ou l'atome d'hydrogène, y₄ représente un nombre entier supérieur ou égal à 2 et inférieur ou égal à 20, v'₁, v'₂, v'₃, identiques ou différents, représentent un nombre entier supérieur ou égal à 0 et inférieur ou égal à 50, et la somme [(y₄. v'₁) + (y₄. v'₂) + v'₃)] est supérieure ou égale à 1 et inférieure ou égale à 50 ;
- Les copolymères polyéthylèneglycol-alkylglycols représentés par la formule (VI) : 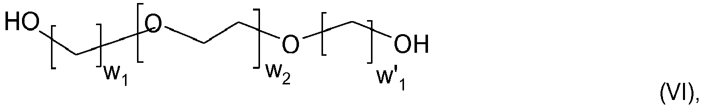 dans laquelle w₁ et w'₁, identiques ou différents, représentent un nombre entier supérieur ou égal à 1 et inférieur ou égal à 50, plus particulièrement supérieur ou égal à 1 et inférieur ou égal à 25, w₂ représente un nombre entier supérieur ou égal à 1 et inférieur ou égal à 100, plus particulièrement supérieur ou égal à 1 et inférieur ou égal à 50.

Parmi les tensioactifs émulsionnants pouvant constituer le système émulsionnant (S₁) compris dans la composition cosmétique (C₃) à usage topique se présentant sous la forme d'une émulsion (E₁₂) de type huile-dans-eau, il y a :
- Les alcools gras et éthoxylés, dont la chaîne grasse alkyle, saturée ou insaturée, linéaire ou ramifiée, comporte de seize à trente-six atomes de carbone et plus particulièrement de douze à vingt-deux atomes de carbone, et dont le degré d'éthoxylation est supérieur à 5 et inférieur ou égal à 200, et plus particulièrement supérieur ou égal à 10 et inférieur ou égal à 100, comme les alcools oléodécylique décaéthoxylé, sous le nom SI-MULSOL™ OC 710, ou laurique heptaéthoxylé commercialisé sous le nom SIMULSOL™ P7 ;
- Les acides gras et éthoxylés, dont la chaîne grasse alkyle, saturée ou insaturée, linéaire ou ramifiée, comporte de huit à trente-six atomes de carbone et plus particulièrement de huit à vingt-deux atomes de carbone, et dont le degré d'éthoxylation est supérieur à 5 et inférieur ou égal à 200, et plus particulièrement supérieur ou égal à 10 et inférieur ou égal à 100 ;

- Les huiles végétales éthoxylées avec une quantité d'oxyde d'éthylène supérieure à 5 moles et inférieure ou égale à 100 moles, plus particulièrement supérieure ou égale à 10 moles et inférieure ou égale à 50 moles, comme l'huile de colza éthoxylée à 10 moles d'oxyde d'éthylène et commercialisée sous le nom SEPITER™C100, l'huile de colza éthoxylée à 20 moles d'oxyde d'éthylène et commercialisée sous le nom SEPITER™C200, l'huile de tournesol éthoxylée à 40 moles d'oxyde d'éthylène et commercialisée sous le nom SEPITER™T400, l'huile de noyaux d'abricots éthoxylée à 40 moles d'oxyde d'éthylène et commercialisée sous le nom SIMULSOL™AO40 ou l'huile de ricin polyéthoxylée avec 40 moles d'oxyde d'éthylène commercialisé sous le nom de marque SIMULSOL™ OL50;
- Les compositions comprenant du stéarate de glycérol et de d'acide stéarique éthoxylé entre 5 moles et 150 moles d'oxyde d'éthylène, comme la composition comprenant de l'acide stéarique éthoxylé à 135 moles d'oxyde d'éthylène et du stéarate de glycérol commercialisée sous le nom SIMULSOL™ 165 ;
- Les esters gras de sorbitan éthoxylés dont la chaîne grasse alkyle, saturée ou insaturée, linéaire ou ramifiée, comporte de huit à dix-huit atomes de carbone, et plus particulièrement de douze à dix-huit atomes de carbone, et dont le degré d'éthoxylation est supérieur ou égal à 5 et inférieur ou égal à 50, plus particulièrement supérieur ou égal à 10 et inférieur ou égal à 25, et encore plus particulièrement égal à 20 comme les produits commercialisés sous le nom de marque MONTANOX™ par la société SEPPIC, les mélanges d'alkylpolyglycosides et d'alcools gras, comme ceux décrits dans les demandes de brevet français 2 668 080, 2 734 496, 2 756 195, 2 762 317, 2 784 680, 2 784 904, 2 791 565, 2 790 977, 2 807 435, 2 804 432, 2 830 774, 2 830 445, et commercialisés sous les noms MONTANOV™ 68, MONTANOV™ 82, MONTANOV™ 202, MONTANOV™ L et MONTANOV™ 14.

Lorsque la composition cosmétique (C₃) à usage topique se présente sous la forme d'une émulsion (E₁), ladite émulsion (E₁) comprend également des excipients et/ou des principes actifs habituellement mis en oeuvre dans le domaine des formulations à usage topique, en particulier cosmétiques, dermocosmétiques, pharmaceutiques ou dermo-pharmaceutiques, comme les tensioactifs moussants et/ou détergents, les tensioactifs épaississants et/ou gélifiants, les agents épaississants et/ou gélifiants, les agents stabilisants, les composés filmogènes, les agents opacificants, les agents nacrants, les agents surgraissants, les séquestrants, les agents conservateurs, les agents conditionneurs, les agents déodorants, les agents blanchissants destinés à la décoloration des poils et de la peau, les principes actifs destinés à apporter une action traitante et/ou protectrice vis à vis de la peau ou des cheveux, les filtres solaires, les charges minérales ou les pigments, les particules procurant un effet visuel ou destinées à l'encapsulation d'actifs, les particules exfoliantes, les agents de texture, les azurants optiques, les répulsifs d'insectes.

Comme exemples de tensioactifs moussants et/ou détergents, éventuellement présents dans la composition (C₃) à usage topique se présentant sous la forme d'une émulsion (E₁), il y a les tensioactifs moussants et/ou détergents anioniques, cationiques, amphotères ou non ioniques topiquement acceptables habituellement utilisés dans ce domaine d'activité.

Parmi les tensioactifs anioniques moussants et/ou détergents que l'on peut associer à la composition (C₃) à usage topique se présentant sous la forme d'une émulsion (E₁), il y a les sels de métaux alcalins, les sels de métaux alcalino-terreux, les sels d'ammonium, les sels d'amines, les sels d'aminoalcools d'alkyléthers sulfates, d'alkylsulfates, d'alkylamidoéthersulfates, d'alkylarylpolyéthersulfates, de monoglycérides sulfates, d'a-oléfinesulfonates, de paraffines sulfonates, d'alkylphosphates, d'alkylétherphosphates, d'alkylsulfonates, d'alkylamidesulfonates, d'alkylarylsulfonates, d'alkylcarboxylates, d'alkylsulfosuccinates, d'alkyléthersulfosuccinates, d'alkylamidesulfosuccinates, d'alkylsulfoacétates, d'alkylsarcosinates, d'acyliséthionates, de N-acyltaurates, d'acyllactylates, de dérivés N-acylés d'acides aminés, de dérivés N-acylés de peptides, de dérivés N-acylés de protéines, d'acides gras.

Parmi les tensioactifs amphotères moussants et/ou détergents que l'on peut associer à la composition (C₃) à usage topique se présentant sous la forme d'une émulsion (E₁), il y a les alkylbétaines, les alkylamidobétaines, les sultaines, les alkylamidoalkylsulfobétaines, les dérivés d'imidazolines, les phosphobétaïnes, les amphopolyacétates et les amphopropionates.

Parmi les tensioactifs cationiques moussants et/ou détergents que l'on peut associer à la composition (C₃) à usage topique se présentant sous la forme d'une émulsion (E₁), il y a particulièrement les dérivés d'ammoniums quaternaires.

Parmi les tensioactifs non ioniques moussants et/ou détergents que l'on peut associer à la composition (C₃) à usage topique se présentant sous la forme d'une émulsion (E₁), il y a plus particulièrement les alkylpolyglycosides comportant un radical aliphatique, linéaire ou ramifié, saturé ou insaturé, et comportant de 8 à 12 atomes de carbone , les amides de coprah, les N-alkylamines.

Parmi les tensioactifs épaississants et/ou gélifiants que l'on peut associer à la composition (C₃) à usage topique se présentant sous la forme d'une émulsion (E₁), il y a les esters gras d'alkylpolyglycosides éventuellement alcoxylés, et tout particulièrement les esters de méthylpolyglucoside éthoxylés tels que le PEG 120 méthyl glucose trioléate et le PEG 120 méthyl glucose dioléate commercialisés respectivement sous les appellations

GLUCAMATE™ LT et GLUMATE™ DOE120 ; les esters gras alcoxylés tels que le PEG 150 pentaérythrytyl tétrastéarate commercialisé sous l'appellation CROTHIX™ DS53, le PEG 55 propylene glycol oléate commercialisé sous l'appellation ANTIL™ 141 ; les carbamates de polyalkylène glycols à chaînes grasses tels que le PPG 14 laureth isophoryl dicarbamate commercialisé sous l'appellation ELFACOS™ T211, le PPG 14 palmeth 60 hexyl dicarbamate commercialisé sous l'appellation ELFACOS™ GT2125.

Parmi les agents épaississants et/ou gélifiants que l'on peut associer à la composition (C₃) à usage topique se présentant sous la forme d'une émulsion (E₁), il y a les agents épaississants et/ou gélifiants décrits précédemment pour la préparation d'une composition cosmétique (C₃) à usage topique se présentant sous la forme d'un gel aqueux (GA).

Parmi les agents opacifiants et/ou nacrants que l'on peut associer à la composition (C₃) à usage topique se présentant sous la forme d'une émulsion (E₁), il y a le palmitate de sodium, le stéarate de sodium, l'hydroxystéarate de sodium, le palmitate de magnésium, le stéarate de magnésium, l'hydroxystéarate de magnésium, le monostéarate d'éthylène glycol, le distéarate d'éthylène glycol, le monostéarate de polyéthylène glycol, le distéarate de polyéthylène glycol, les alcools gras comportant de 12 à 22 atomes de carbone.

Parmi les principes actifs que l'on peut associer à la composition (C₃) à usage topique se présentant sous la forme d'une émulsion (E₁), il y a par exemple : les vitamines et leurs dérives, notamment leurs esters, tels que le rétinol (vitamine A) et ses esters (palmitate de rétinyle par exemple), l'acide ascorbique (vitamine C) et ses esters, les dérives de sucre de l'acide ascorbique (comme l'ascorbyl glucoside), le tocophérol (vitamine E) et ses esters (comme l'acétate de tocophérol), les vitamines B3 ou B10 (niacinamide et ses dérivés) ; les composés montrant une action éclaircissante ou dépigmentante de la peau comme le SEPIWHITE™MSH, l'arbutine, l'acide kojique, l'hydroquinone, le VEGEWHITE™, la GATULINE™, le SYNERLIGHT™, le BIOWHITE™, le PHYTOLIGHT™, le DERMALIGHT™, la CLARISKIN™, le MELASLOW™, le DERMAWHITE™, l'ETHIOLINE, le MELAREST™, le GIGAWHITE™, l'ALBATINE™, le LUMISKIN™ ; les composés montrant une action apaisante comme le SEPICALM™ S, l'allantoïne et le bisabolol ; les agents anti-inflammatoires ; les composés montrant une action hydratante comme l'urée, les hydroxyurées, le glycérol, les polyglycérols, le glycérolglucoside, le diglycérolglucoside, les polyglycérylglucosides ; les extraits végétaux riches en polyphénols comme les extraits de raisin, les extraits de pin, les extraits de vin, les extraits d'olives ; les composés montrant une action amincissante ou lipolytique comme la caféine ou ses dérivés, l'ADIPOSLIM™, l'ADIPOLESS™ ; les protéines N-acylées ; les peptides N-acylés comme le MATRIXIL™ ; les acides aminés N-acylés ; les hydrolysâts partiels de protéines N-acylés ; les acides aminés ; les peptides ; les hydrolysâts totaux de protéines ; les extraits de soja, par exemple la Raffermine™ ; les extraits de blé par exemple la TENSINE™ ou la GLIADINE™ ; les extraits végétaux riches en tanins ; les extraits végétaux riches en isoflavones ; les extraits végétaux riches en terpènes ; les extraits d'algues d'eaux douces, d'eaux marines, d'eaux saumâtres ; les extraits de plantes marines halphytes ; les extraits marins en général comme les coraux ; les extraits bactériens ; les céramides ; les phospholipides ; les composés montrant une action antimicrobienne ou une action purifiante, comme le LIPACIDE™ C8G, le LIPACIDE™ UG, le SEPICONTROL™ A5 ; l'OCTOPIROX™ ou le SENSIVA™ SC50 ; les composés montrant une propriété énergisante ou stimulante comme le Physiogényl™, le panthénol et ses dérivés comme le SEPICAP™ MP ; les actifs anti-âge comme le SEPILIFT™ DPHP, le LIPACIDE™ PVB, le SEPIVINOL™, le SEPIVITAL™, le MANOLIVA™ le PHYTO-AGE™, le TIMECODE™ ; le SURVICODE™ ; les actifs anti-photo vieillissement ; les actifs protecteurs de l'intégrité de la jonction dermo-épidermique ; les actifs augmentant la synthèse des composants de la matrice extracellulaire comme le collagène, les élastines, les glycosaminoglycanes ; les actifs agissant favorablement sur la communication cellulaire chimique comme les cytokines ou physiques comme les intégrines ; les actifs créant une sensation de « chauffe » sur la peau comme les activateurs de la microcirculation cutanée (comme les dérivés de l'acide nicotinique) ou des produits créant une sensation de « fraîcheur » sur la peau (comme le menthol et des dérivés) ; les actifs améliorant la microcirculation cutanée, par exemple les veinotoniques ; les actifs drainants ; les actifs à visée décongestionnante comme les extraits de ginko biloba, de lierre, de marron d'inde, de bambou, de ruscus, de petit houx, de *centalla asiatica,* de fucus, de romarin, de saule ; les agents de bronzage ou de brunissement de la peau, comme la dihydroxyacétone, l'isatine, l'alloxane, la ninhydrine, le glycéraldéhyde, l'aldéhyde mésotartrique, la glutaraldéhyde, l'érythrulose.

Parmi les agents déodorants que l'on peut associer à la composition (C₃) à usage topique se présentant sous la forme d'une émulsion (E₁), il y a par exemple les silicates alcalins, les sels de zinc comme le sulfate de zinc, le gluconate de zinc, le chlorure de zinc, le lactate de zinc ; les sels d'ammonium quaternaires comme les sels de cétyltriméthylammonium, les sels de cétylpyridinium ; les dérivés du glycérol comme le caprate de glycérol , le caprylate de glycérol, le caprate de polyglycérol ; le 1,2 décanediol ; le 1,3 propanediol ; l'acide salicylique ; le bicarbonate de sodium; les cyclodextrines ; les zéolithes métalliques ; le Triclosan™ ; le bromohydrate d'aluinium, les chlorhydrates d'aluminium, le chlorure d'aluminium, le sulfate d'aluminium, les chlorhydrates d'aluminium et de zirconium, le trichlorhydrate d'aluminium et de zirconium, le tétrachlorhydrate d'aluminium et de zirconium, le pentachlorhydrate d'aluminium et de zirconium, l'octochlorhydrate d'aluminium et de zirconium, le sulfate d'aluminium, le lactate de sodium et d'aluminium, les complexes de chlorhydrate d'aluminium et de glycol, comme le complexe de chlorhydrate d'aluminium et de propylène glycol, le complexe de dichlorhydrate d'aluminium et de propylène glycol, le complexe de sesquichlorhydrate d'aluminium et de propylène glycol, le complexe de chlorhydrate d'aluminium et de polyéthylène glycol, le complexe de dichlorhydrate d'aluminium et de polyéthylène glycol, le complexe de sesquichlorhydrate d'aluminium et de polyéthylène glycol.
Parmi les filtres solaires que l'on peut associer à la composition (C₃) à usage topique se présentant sous la forme d'une émulsion (E₁), il y a tous ceux figurant dans la directive cosmétique 76/768/CEE modifiée annexe VII.

Lorsque la composition cosmétique (C₃) à usage topique se présente sous la forme d'une émulsion (E₁₂) de type huile-dans-eau, ladite émulsion (E₁₂) comprend généralement :
- De 5% à 55% massique, plus particulièrement de 7% à 30% massique, et encore plus particulièrement de 10% à 20% massique d'une phase grasse (P1) constituée d'au moins une huile et/ou d'au moins une cire telle que décrite précédemment,
- De 95% à 45% massique, plus particulièrement de 93% à 70% massique, et encore plus particulièrement de 90% à 80% massique de la solution aqueuse (C₂) telle que définie précédemment.

Lorsque la composition cosmétique (C₃) à usage topique se présente sous la forme d'une émulsion (E₁₁) de type eau-dans-huile, ladite émulsion (E₁₁) comprend généralement :
- De 5% à 70% massique, plus particulièrement de 10% à 60% massique, et encore plus particulièrement de 20% à 60% massique de la solution aqueuse (C₂) telle que définie précédemment,
- De 95% à 30% massique, plus particulièrement de 90% à 40% massique, et encore plus particulièrement de 80% à 40% massique d'une phase grasse (P1) constituée d'au moins une huile et/ou d'au moins une cire telle que décrite précédemment.

L'étude expérimentale suivante illustre l'invention.

### 1) Préparation de compositions représentées par la formule (I) et évaluation de leurs propriétés solubilisantes selon l'invention

### 1.1 Préparation d'une composition de n-heptyl polyglucosides

On introduit 2,7 équivalents molaires de n-heptanol dans un réacteur en verre à double enveloppe, dans laquelle circule un fluide caloporteur, et muni d'une agitation efficace, à une température de 40°C. Un équivalent molaire de glucose anhydre est alors ajouté progressivement au milieu réactionnel pour permettre sa dispersion homogène, puis 0,15 % massique d'acide sulfurique à 98% et 0,15 % massique d'acide hypophosphoreux à 50% pour 100% de la masse constituée par la somme de la massse du glucose et de la masse du n-heptanol sont introduits dans la dispersion homogène préalablement préparée. Le milieu réactionnel est placé sous un vide partiel d'environ 180 mbars, et maintenu à une température de 100°C-105°C pendant une durée de 4 heures avec évacuation de l'eau formée au moyen d'un montage de distillation. Le milieu réactionnel est ensuite refroidi à 85°C-90°C et neutralisé par ajout de soude à 40 %, pour amener le pH d'une solution à 5 % de ce mélange à une valeur d'environ 7,0. La milieu réactionnel ainsi obtenu est ensuite vidangée à une température de 70°C et filtré pour éliminer les grains de glucose n'ayant pas réagi. Le filtrat est ensuite introduit dans un réacteur en verre à double enveloppe, dans laquelle circule un fluide caloporteur, muni d'une agitation efficace et d'un dispositif de distillation. L'excès d'heptanol est ensuite éliminé par distillation à une température de 120°C sous vide partiel compris entre environ 100 mbars et 50 mbars. Le milieu réactionnel ainsi distillé est aussitôt dilué par l'ajout d'une quantité d'eau de façon à atteindre une concentration en milieu réactionnel d'environ 60%. Après homogénéisation pendant 30 minutes à une température de 50°C, la composition (X₁) obtenue est vidangée.

Les caractéristiques analytiques de la composition (X₁) ainsi obtenue comprenant des n-heptylpolyglucosides sont consignés dans le tableau 1 ci-dessous.

**Tableau 1**

| | Composition (X₁) |
|---|---|
| Aspect à 20°C (Détermination visuelle) | Liquide |
| Indice d'acide (Norme NFT 60204) | 1,7 |
| Indice d'hydroxyle sur extrait sec (Norme USP XXI NF XVI 01/01/1995) | 813,9 |
| Eau (% massique) (Norme NFT 73201) | 58,8 % |
| Teneur résiduelle en n-heptanol (chromatographie en phase gazeuse) en % massique | 0,22 % |

### 2) Préparation de compositions comparatives.

### 2.1 Préparation d'une composition de n-pentyl polyglucosides

On introduit 3,1 équivalents molaires de n-pentanol dans un réacteur en verre à double enveloppe, dans laquelle circule un fluide caloporteur, et muni d'une agitation efficace, à une température de 105°C. Un équivalent molaire de glucose anhydre est alors ajouté progressivement au milieu réactionnel pour permettre sa dispersion homogène, puis 0,14 % massique d'acide sulfurique à 98% et 0,14 % massique d'acide hypophosphoreux à 50% pour 100% de la masse constituée par la somme de la massse du glucose et de la masse du n-pentanol sont introduits dans la dispersion homogène préalablement préparée. Le milieu réactionnel est placé sous un vide partiel d'environ 400 mbars, et maintenu à une température de 100°C-105°C pendant une durée de 6 heures avec évacuation de l'eau formée au moyen d'un montage de distillation. Le milieu réactionnel est ensuite refroidi à 85°C-90°C et neutralisé par ajout de soude à 40 %, pour amener le pH d'une solution à 5 % de ce mélange à une valeur d'environ 7,0. La milieu réactionnel ainsi obtenu est ensuite vidangée à une température de 70 °C et filtré pour éliminer les grains de glucose n'ayant pas réagi. Le filtrat est ensuite introduit dans un réacteur en verre à double enveloppe, dans laquelle circule un fluide caloporteur, muni d'une agitation efficace et d'un dispositif de distillation. L'excès de n-pentanol est ensuite éliminé par distillation à une température de 110 °C sous vide partiel compris entre environ 125 mbars et 50 mbars. Le milieu réactionnel ainsi distillé est aussitôt dilué par l'ajout d'une quantité d'eau de façon à atteindre une concentration en milieu réactionnel d'environ 70 %. Après homogénéisation pendant 60 minutes à une température de 40 °C, la composition (X₅) obtenue est vidangée.

**Tableau 2**

| | Composition (X₅) |
|---|---|
| Aspect à 20°C (Détermination visuelle) | Liquide lipide orange |
| Indice d'acide (Norme NFT 60204) | 0,7 |
| Indice d'hydroxyle sur extrait sec (Norme USP XXI NF XVI 01/01/1995) | 840 |
| pH à 5% (Norme NFT 73206) | 5,5 |
| Eau (% massique) (Norme NFT 73201) | 28,1 % |
| Teneur résiduelle en n-pentanol (chromatographie en phase gazeuse) en % massique | 0,2 % |

### 2.2 Préparation d'une composition (X₆) comprenant une composition de n-pentyl polyglucosides et une composition de caprylyl/caprylic polyglucoside (50/50)

Dans un réacteur en verre à double enveloppe d'une capacité de deux litres, dans laquelle circule un fluide caloporteur, et muni d'une agitation efficace, à une température de 25°C, on introduit 100 grammes de la composition (X₅) obtenue selon le procédé décrit dans la section 2.2 de la présente demande, et 118,8 grammes d'une composition de caprylyl/caprylic glucoside (50/50) [composition (X₃)], commercialisée sous le nom ORAMIX™ CG110 dont l'extrait sec est de 60,5 %.

Le mélange est agité pendant 30 minutes à 25°C, puis la composition (X₆) ainsi obtenue est vidangée. La composition (X₆) ainsi obtenue comprend, pour 100% de sa masse sèche, 50% massique de n-pentyl polyglucosides et 50% massique de caprylyl/caprylic polyglucoside (50/50).

### 3) Evaluation des propriétés solubilisantes de n-heptylpolyglucosides et des compositions comparatives pour solubiliser des huiles essentielles et des parfums dans l'eau.

Les propriétés solubilisantes de la composition (X₁) obtenue selon le procédé décrit au paragraphe précédent, vis-à-vis d'huiles essentielles et de parfums dans l'eau, ont été évaluées comparativement à celles de la composition de (2-éthyl hexyl) polyglucosides, commercialisée sous le nom AG™ 6202 [composition (X₂)], à celles d'une composition de caprylyl/caprylic polyglucoside (50/50) [composition (X₃)], commercialisée sous le nom ORAMIX™ CG110, à celles d'une composition d'huile de ricin hydrogénée et éthoxylée à 40 moles d'oxyde d'éthylène [composition (X₄)], dont le nom INCI est « PEG-40 hydroge-nated castor oil » et dont le numéro CAS est 61788-85-0, commercialisée sous l'appellation SIMULSOL™1293, à celles de la composition (X₅) et à celle de composition (X₆) selon la méthode d'évaluation décrite ci-dessous.

### 3.1- Protocole expérimental

Pour chaque « substance parfumante » hydrophobe (huile essentielle ou parfum) à solubiliser, on introduit dans un flacon de verre de 150 cm³ un gramme en matière sèche de ladite substance parfumante hydrophobe et une quantité Yᵢ grammes de composition tensioactive à évaluer comme agent solubilisant. Une quantité d'eau distillée est ajoutée à une température de 20°C de manière complémentaire de façon à obtenir une masse totale de cent grammes, de sorte à ce que le mélange final comprenne 1% de substance parfumante hydrophobe à solubiliser et Yᵢ% massique d'agent solubilisant. La température est régulée à 20°C. Un barreau magnétique aimanté est introduit dans le flacon de verre qui est ensuite placé sous agitation magnétique à une vitesse de 80 tours/min pendant une durée de 60 minutes à une température (T₁) de 20°C. A l'issue de cette durée, on note l'aspect visuel obtenu. On détermine ainsi, pour chaque « substance parfumante » hydrophobe, la quantité massique minimale requise pour solubiliser 1% massique de ladite « substance parfumante » hydrophobe ; et on mesure, pour cette quantité minimale d'agent solubilisant, la turbidité de la solution obtenue à une température de 20°C, à l'aide d'un turbidimètre optique de marque HF Scientific et de modèle DRT100B, préalablement étalonné avec une solution de Formazine (0,9 NTU). Ce protocole expérimental a été mis en oeuvre pour chacune des compositions (X₁) selon l'invention et (X₂), (X₃), (X₄), (X₅) et (X₆) selon l'état de la technique.

### 3.2 - Expression des résultats

Les aspects visuels de chacune des solutions obtenues selon le protocole de la section 1.2.1 de la présente demande sont notés par l'expérimentateur et qualifiés de limpide (L), de trouble (T) ou de trouble - hétérogène (T-H), selon les cas.
Les mesures de turbidité sont exprimées en unité NTU.

### 3.3- Caractérisation de la solubilisation en milieu aqueux, à une température de 20°C de substances parfumantes hydrophobes par la composition (X₁) selon l'invention en comparaison avec les compositions (X₂), (X₃), (X₄), (X₅) et (X₆) de l'état de la technique.

Les aspects des solutions préparées pour chacune des compositions (X₁) selon l'invention et (X₂), (X₃), (X₄), (X₅) et (X₆) selon l'état de la technique, utilisée pour solubiliser les substances parfumantes à 20°C ont été relevés par l'expérimentateur et consignés respectivement dans les tableaux ci-dessous, pour des huiles essentielles de clou de girofle, de Palmarosa, de Cabreuva ainsi que pour un parfum de type « accord floral » référencé sous le nom « Just delicious » par la société Technicolor.

**Tableau 3.1 : Aspect de solutions comprenant 1% massique d'huile essentielle de clou de girofle et une quantité variable de (X₁), (X₂), (X₃), (X₄), (X₅) ou (X₆)**

| | Aspect de la solution | | |
|---|---|---|---|
| Fraction massique en (X₁), (X₂) ou (X₃) (% en matière sèche) | (X₁) | (X₂) | (X₃) |
| 0% | T-H | T-H | T-H |
| 1,0% | T | T | T |
| 2,0% | L | T | T |
| 3,0% | L | T | T |
| 4,0% | n.m. | T | T |
| 5,0% | n.m. | T | T |
| 6,0% | n.m. | T | L |
| 7,0% | n.m. | T | L |
| 8,0% | n.m. | L | n.m. |
| 9,0% | n.m. | L | n.m. |

| Fraction massique en (X₄), (X₅) ou (X₆) (% en matière sèche) | (X₄) | (X₅) | (X₆) |
|---|---|---|---|
| 0% | T-H | T-H | T-H |
| 1,0% | T | T | T |
| 2,0% | T | T | T |
| 3,0% | T | T | T |
| 4,0% | T | T | T |
| 5,0% | T | T | T |
| 6,0% | T | T | T |
| 7,0% | L | T | T |
| 8,0% | L | T | L |
| 9,0% | n.m. | n.m. | n.m. |

| | | | |
|---|---|---|---|
| (*) n.m. : non mesurée | | | |

**Tableau 3.2 : Ratio massique minimal : huile essentielle de clou de girofle / composition tensioactive Xi (Rₘᵢₙ) pour obtenir une solution limpide.**

| Composition | (X₁) | (X₂) | (X₃) | (X₄) | (X₅) | (X₆) |
|---|---|---|---|---|---|---|
| Rₘᵢₙᵢ | 1/2 | 1/8 | 1/6 | 1/7 | 1/8 | 1/8 |
| Turbidité (NTU) | 3,6 | 4,8 | 27,3 | 18,5 | Insoluble | 9,8 |

**Tableau 4.1 : Aspect de solution comprenant 1% massique d'huile essentielle de Palmarosa et une quantité variable de (X₁), (X₂), (X₃), (X₄), (X₅) ou (X₆)**

| | Aspect de la solution | | |
|---|---|---|---|
| Fraction massique en (X₁), (X₂) ou (X₃) (% en matière sèche) | (X₁) | (X₂) | (X₃) |
| 0% | T-H | T-H | T-H |
| 1,0% | T | T | T |
| 2,0% | T | T | T |
| 3,0% | T | T | T |
| 4,0% | T | T | T |
| 5,0% | T | T | T |
| 6,0% | L | T | T |
| 7,0% | L | T | T |
| 8,0% | n.m. | T | L |
| 9,0% | n.m. | L | L |

| Fraction massique en (X₄), (X₅) ou (X₆) (% en matière sèche) | (X₄) | (X₅) | (X₆) |
|---|---|---|---|
| 0% | T-H | T-H | T-H |
| 1,0% | T | T | T |
| 2,0% | T | T | T |
| 3,0% | T | T | T |
| 4,0% | T | T | T |
| 5,0% | T | T | T |
| 6,0% | T | T | T |
| 7,0% | T | T | T |
| 8,0% | T | T | T |
| 9,0% | L | n.m. | n.m. |

| | | | |
|---|---|---|---|
| (*) n.m. : non mesurée | | | |

**Tableau 4.2 : Ratio massique minimal : huile essentielle de Palmarosa / composition tensioactive Xi (Rₘᵢₙ), pour obtenir une solution limpide.**

| Composition | (X₁) | (X₂) | (X₃) | (X₄) | (X₅) | (X₆) |
|---|---|---|---|---|---|---|
| Rₘᵢₙᵢ | 1/6 | 1/9 | 1/8 | 1/9 | 1/8 | 1/8 |
| Turbidité (NTU) | 11 | 5,2 | 25,5 | Insoluble | Insoluble | Insoluble |

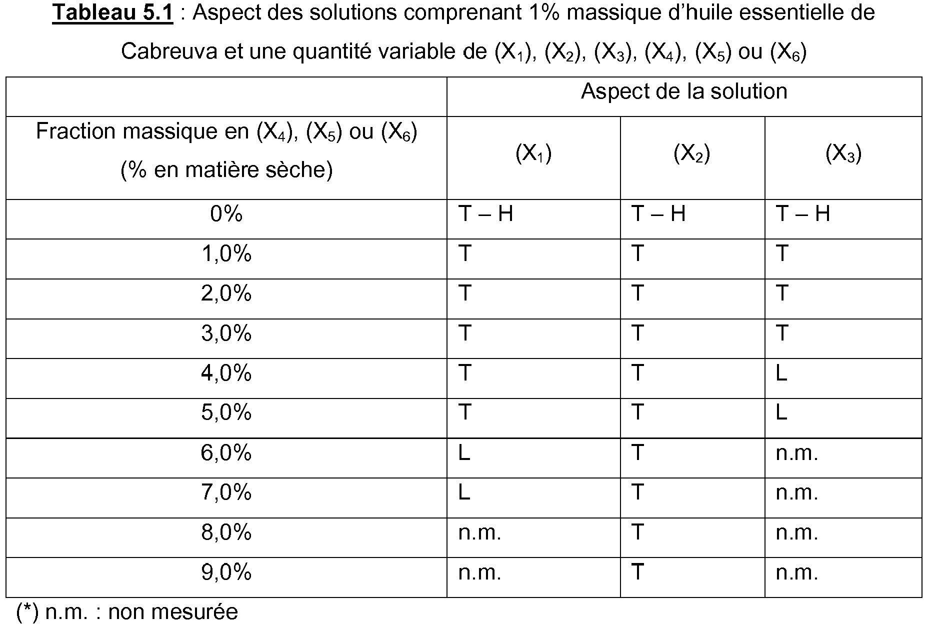

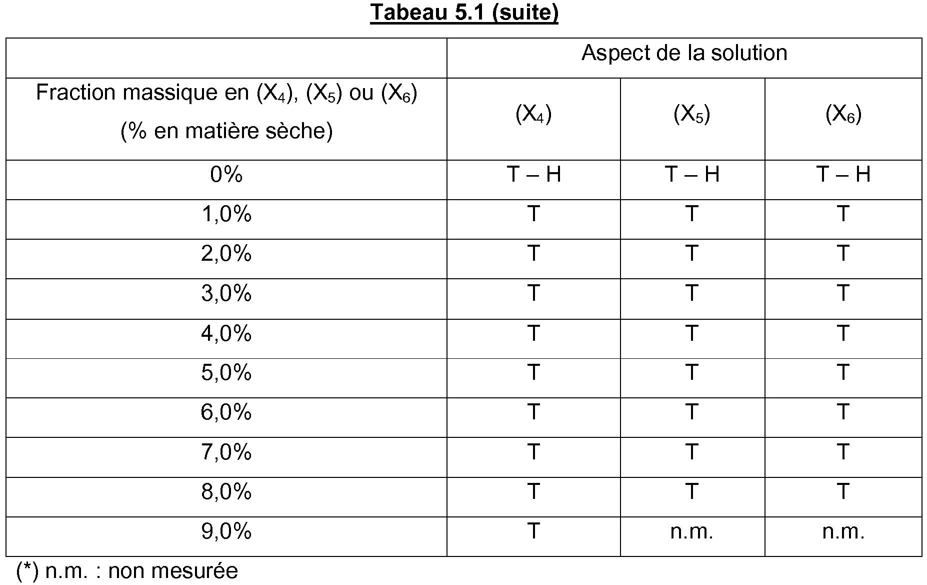

**Tableau 5.2 : Ratio massique minimal : huile essentielle de Cabreuva / composition tensioactive Xi (Rₘᵢₙ) pour obtenir une solution limpide.**

| Composition | (X₁) | (X₂) | (X₃) | (X₄) | (X₅) | (X₆) |
|---|---|---|---|---|---|---|
| Rₘᵢₙᵢ | 1/6 | 1/9 | 1/3 | 1/9 | 1/8 | 1/8 |
| Turbidité (NTU) | 3,8 | Insoluble | 857 | Insoluble | Insoluble | Insoluble |

### 3.4 Analyse des résultats.

La solubilisation d'huile essentielle de clou de girofle nécessite une quantité de la composition (X₁) plus faible que celles des compositions (X₂), (X₃), (X₄), (X₅) et (X₆).

La composition (X₁) comprenant des n-heptyl polyglucosides permet de solubiliser dans l'eau l'huile essentielle de clou de girofle dans un ratio massique huile essentielle/composition (X₁) de 1/2 alors que les compositions solubilisantes comparatives nécessitent d'être employée dans une quantité massique supérieure, à savoir dans un ratio huile essentielle/composition (X₂) de 1/8, dans un ratio huile essentielle/composition (X₃) de 1/6, dans un ratio huile essentielle/composition (X₄) de 1/7, dans un ratio huile essentielle/ composition (X₆) de 1/8 ; la composition (X₅) ne permet pas de solubiliser l'huile essentielle de clou de girofle dans un ratio huile essentielle/composition (X₅) inférieur ou égal à 1/8.

La composition (X₁) comprenant des n-heptyl polyglucosides permet de solubiliser dans l'eau l'huile essentielle de palmarosa dans un ratio massique huile essentielle/composition (X₁) de 1/6 alors que les compositions solubilisantes comparatives nécessitent d'être employée dans une quantité massique supérieure, à savoir dans un ratio huile essentielle/composition (X₂) de 1/9, dans un ratio huile essentielle/composition (X₃) de 1/8 et dans un ratio huile essentielle/composition (X₄) de 1/9. Les compositions (X₅) et (X₆) ne permettent pas de solubiliser l'huile essentielle de palmarosa dans l'eau dans un ratio huile essentielle/composition (X₅) et dans un ratio huile essentielle/composition (X₆) inférieur ou égal à 1/8.

La composition (X₁) comprenant des n-heptyl polyglucosides permet de solubiliser dans l'eau l'huile essentielle de cabreuva dans un ratio massique huile essentielle/composition (X₁) de 1/6 alors que les compositions solubilisantes comparatives nécessitent d'être employée dans une quantité massique supérieure, à savoir dans un ratio huile essentielle/composition (X₂) supérieure à 1/9, dans un ratio huile essentielle/composition (X₃) de 1/4 et dans un ratio huile essentielle/composition (X₄) supérieure à 1/9 ; la composition (X₃) présentant cependant l'inconvénient de générer la formation de mousse lors de l'agitation de la solution la comprenant, phénomène non observé avec la composition (X₁). Les compositions (X₅) et (X₆) ne permettent pas de solubiliser l'huile essentielle de cabreuva dans l'eau dans un ratio huile essentielle/composition (X₅) et dans un ratio huile essentielle/composition (X₆) inférieur ou égal à 1/8.

La composition (X₁) comprenant des n-heptyl polyglucosides permet également d'obtenir une solution aqueuse comprenant 1% d'huile essentielle de clou de girofle et une solution aqueuse d'huile essentielle de Cabreuva plus transparente, à savoir 3,6 NTU pour la solution comprenant l'huile essentielle de clou de girofle et 3,8 NTU pour la solution comprenant l'huile essentielle de Cabreuva, que les compositions solubilisantes comparatives testées avec ces mêmes huiles essentielles.

## Revendications

1. Utilisation d'une composition représentée par la formule (I) :
R-O-(G)ₚ-H (I),
dans laquelle R représente le radical n-heptyle, G représente le reste du glucose et p représente un nombre décimal supérieur ou égal à 1,05 et inférieur ou égal à 2,5, ladite composition de formule (I) consistant en un mélange de composés représentés par les formules (I₁), (I₂), (I₃), (I₄) et (I₅) :
R-O-(G)₁-H (I₁)
R-O-(G)₂-H (I₂)
R-O-(G)₃-H (I₃)
R-O-(G)₄-H (I₄)
R-O-(G)₅-H (I₅)
dans les proportions molaires respectives a₁, a₂, a₃, a₄ et a₅, telles que chacune des proportions a₁, a₂, a₃, a₄ et a₅ est supérieure ou égale à zéro et inférieure ou égale à un et que la somme a₁ + a₂ + a₃ + a₄ + a₅ est égale à un, comme agent solubilisant en solution aqueuse, d'une substance parfumante hydrophobe (A) ou d'un mélange de substances parfumantes hydrophobes (A), **caractérisée en ce que** ladite substance parfumante hydrophobe (A) est sélectionnée dans le groupe constitué des huiles essentielles riches en composés portant une fonction phénolique, lesdits composés portant une fonction phénolique étant sélectionnés dans le groupe constitué du thymol, du carvacol de l'eugénol, de l'iso-eugénol et de l'estragol, et des huiles essentielles riches en composés portant une fonction alcool, lesdits composés portant une fonction alcool étant sélectionnés dans le groupe constitué du linalol, du géraniol, du menthol, du néomenthol, du cis-hydrate de sabinène, du citronellol, de l'a-terpinéol, du viridiflorol, du trans-nérolidol, de l'a-cadinol, du τ-cadinol, du trans-farnesol, du cis-farnesol, de l'a-bisabolol, du patchoulol et du pogostol.

2. Utilisation telle que définie à la revendication 1, **caractérisée en ce que** le rapport massique entre ladite substance parfumante hydrophobe (A) et la composition représentée par la formule (I) est supérieur ou égal à 1/7 et inférieur ou égal à 2/1.

3. Solution aqueuse (C₂) comprenant pour 100% de sa masse :
**a)** - de 0,05 % à 70 % massique d'au moins une composition représentée par la formule (I) :
R-O-(G)ₚ-H (I)
dans laquelle R représente le radical n-heptyle, G représente le reste du glucose et p représente un nombre décimal supérieur ou égal à 1,05 et inférieur ou égal à 2,5, ladite composition de formule (I) consistant en un mélange de composés représentés par les formules (I₁), (I₂), (I₃), (I₄) et (I₅) :
R-O-(G)₁-H (I₁)
R-O-(G)₂-H (I₂)
R-O-(G)₃-H (I₃)
R-O-(G)₄-H (I₄)
R-O-(G)₅-H (I₅)
dans les proportions molaires respectives a₁, a₂, a₃, a₄ et a₅, telles que la somme a₁ + a₂ + a₃ + a₄ + a₅ est égale à un et que chacune des proportions a₁, a₂, a₃, a₄ et a₅ est supérieure ou égale à zéro et inférieure ou égale à un ;
**b)** - de 0,1% à 10% massique d'une huile essentielle riche en composés portant une fonction phénolique ou portant une fonction alcool, lesdits composés portant une fonction phénolique étant sélectionnés dans le groupe constitué du thymol, du carvacol de l'eugénol, de l'iso-eugénol et de l'estragol, et lesdits composés portant une fonction alcool étant sélectionnés dans le groupe constitué du linalol, du géraniol, du menthol, du néomenthol, du cis-hydrate de sabinène, du citronellol, de l'α-terpinéol, du viridiflorol, du trans-nérolidol, de l'a-cadinol, du τ-cadinol, du trans-farnesol, du cis-farnesol, de l'a-bisabolol, du patchoulol et du pogostol, ou d'un mélange de ces huiles ;
**c)** - de 20 % à 99,85 % massique d'eau.

4. Solution aqueuse (C₂) telle que définie à la revendication 3, **caractérisée en ce que** le rapport massique entre ladite substance parfumante hydrophobe (A) et la composition représentée par la formule (I) est supérieur ou égal à 1/7 et inférieur ou égal à 2/1.

5. Solution aqueuse (C₂) telle que définie à l'une quelconque des revendications 3 ou 4, **caractérisée en ce qu'**elle présente une turbidité supérieure ou égale à 1 NTU et inférieure ou égale à 100 NTU, mesurée quantitativement par une méthode optique néphélométrique.

6. Procédé pour parfumer la peau, les cheveux, le cuir chevelu, les muqueuses ou un vêtement **caractérisé en ce qu'**il comprend une étape d'application sur ladite peau, lesdits cheveux, ledit cuir chevelu, lesdites muqueuses ou ledit vêtement de la solution aqueuse (C₂) telle que définie à l'une quelconque des revendications 4 à 6.

7. Utilisation de la solution aqueuse (C₂) telle que définie à l'une quelconque des revendications 4 à 6, comme agent parfumant pour préparer une composition cosmétique (C₃) à usage topique.

## Patentansprüche

1. Verwendung einer durch die Formel (I) dargestellten Zusammensetzung:
R-O-(G)ₚ-H (I),
wobei R den n-Heptylrest darstellt, G den Glucoserest darstellt und p eine Dezimalzahl größer als oder gleich 1,05 und kleiner als oder gleich 2,5 darstellt, wobei die Zusammensetzung der Formel (I) aus einem Gemisch von Verbindungen besteht, dargestellt durch die Formeln (I₁), (I₂), (I₃), (I₄) und (I₅):
R-O-(G)₁-H (I₁)
R-O-(G)₂-H (I₂)
R-O-(G)₃-H (I₃)
R-O-(G)₄-H (I₄)
R-O-(G)₅-H (I₅)
jeweils in molaren Proportionen a₁, a₂, a₃, a₄ und a₅ derart, dass jede der Proportionen a₁, a₂, a₃, a₄ und a₅ größer als oder gleich Null und kleiner als oder gleich Eins ist, und dass die Summe a₁ + a₂ + a₃ + a₄ + a₅ gleich Eins ist, als Lösemittel in wässriger Lösung, einer hydrophoben parfümierenden Substanz (A) oder eines Gemischs von hydrophoben parfümierenden Substanzen (A), **dadurch gekennzeichnet, dass** die hydrophobe parfümierende Substanz (A) ausgewählt ist aus der Gruppe, bestehend aus ätherischen Ölen reich an Verbindungen, die eine Phenolfunktion tragen, wobei die Verbindungen, die eine Phenolfunktion tragen, ausgewählt sind aus der Gruppe, bestehend aus Thymol, Carvacol, Eugenol, Isoeugenol und Estragol, und ätherischen Ölen reich an Verbindungen, die eine Alkoholfunktion tragen, wobei die Verbindungen, die eine Alkoholfunktion tragen, ausgewählt sind aus der Gruppe, bestehend aus Linalol, Geraniol, Menthol, Neomenthol, cis-Sabinenhydrat, Citronellol, α-Terpineol, Viridiflorol, trans-Nerolidol, α-Cadinol, T-Cadinol, trans-Farnesol, cis-Farnesol, α-Bisabolol, Patchoulol und Pogostol.

2. Verwendung nach Anspruch 1, **dadurch gekennzeichnet, dass** das Massenverhältnis zwischen der hydrophoben parfümierenden Substanz (A) und der durch die Formel (I) dargestellten Zusammensetzung größer als oder gleich 1/7 und kleiner als oder gleich 2/1 ist.

3. Wässrige Lösung (C2), umfassend für 100 % seiner Masse:
a) - von 0,05 Massen- % bis 70 Massen- % mindestens eine durch die Formel (I) dargestellte Zusammensetzung:
R-O-(G)ₚ-H (I)
wobei R den n-Heptylrest darstellt, G den Glucoserest darstellt und p eine Dezimalzahl größer als oder gleich 1,05 und kleiner als oder gleich 2,5 darstellt, wobei die Zusammensetzung der Formel (I) aus einem Gemisch von Verbindungen besteht, dargestellt durch die Formeln (I₁), (I₂), (I₃), (I₄) und (I₅):
R-O-(G)₁-H (I₁)
R-O-(G)₂-H (I₂)
R-O-(G)₃-H (I₃)
R-O-(G)₄-H (I₄)
R-O-(G)₅-H (I₅)
jeweils in molaren Proportionen a₁, a₂, a₃, a₄ und a₅ derart, dass die Summe a₁ + a₂ + a₃ + a₄ + a₅ gleich Eins ist und jede der Proportionen a₁ + a₂ + a₃ + a₄ + a₅ größer als oder gleich Null und kleiner als oder gleich Eins ist;
b) - von 0,1 Massen- % bis 10 Massen- % ätherisches Öl reich an Verbindungen, die eine Phenolfunktion tragen oder eine Alkoholfunktion tragen, wobei Verbindungen, die eine Phenolfunktion tragen, ausgewählt sind aus der Gruppe, bestehend aus Thymol, Carvacol, Eugenol, Isoeugenol und Estragol, und wobei die Verbindungen, die eine Alkoholfunktion tragen, ausgewählt sind aus der Gruppe, bestehend aus Linalol, Geraniol, Menthol, Neomenthol, cis-Sabinenhydrat, Citronellol, α-Terpineol, Viridiflorol, trans-Nerolidol, α-Cadinol, T-Cadinol, trans-Farnesol, cis-Farnesol, α-Bisabolol, Patchoulol und Pogostol, oder aus einem Gemisch dieser Öle;
c) - von 20 Massen- % bis 99,85 Massen- % Wasser.

4. Wässrige Lösung (C₂) nach Anspruch 3, **dadurch gekennzeichnet, dass** das Massenverhältnis zwischen der hydrophoben parfümierenden Substanz (A) und der durch die Formel (I) dargestellten Zusammensetzung größer als oder gleich 1/7 und kleiner als oder gleich 2/1 ist.

5. Wässrige Lösung (C₂) nach einem der Ansprüche 3 oder 4, **dadurch gekennzeichnet, dass** sie eine Trübung größer als oder gleich 1 NTU und kleiner als oder gleich 100 NTU aufweist, quantitativ gemessen durch ein optisches nephelometrisches Verfahren.

6. Verfahren zum Parfümieren der Haut, der Haare, der Kopfhaut, der Schleimhäute oder eines Kleindungsstücks, **dadurch gekennzeichnet, dass** es einen Auftragungsschritt der wässrigen Lösung (C₂) auf die Haut, die Haare, die Kopfhaut, die Schleimhäute oder das Kleidungsstück nach einem der Ansprüche 4 bis 6 umfasst.

7. Verwendung der wässrigen Lösung (C₂) nach einem der Ansprüche 4 bis 6, als Parfümierungsmittel zum Zubereiten einer kosmetischen Zusammensetzung (C₃) zur topischen Anwendung.

## Claims

1. Use of a composition represented by the formula (I):
R-0-(G)ₚ-H (I),
wherein R represents the n-heptyl radical, G represents the residue of the sugar and p represents a decimal number greater than or equal to 1.05 and less than or equal to 2.5, said composition of formula (I) consisting of a mixture compounds represented by formulas (I₁), (I₂), (I₃), (I₄) and (I₅):
R-O-(G)₁-H (I₁)
R-O-(G)₂-H (I₂)
R-O-(G)₃-H (I₃)
R-O-(G)₄-H (I₄)
R-O-(G)₅-H (I₅)
in the respective molar proportions a₁, a₂, a₃, a₄, a₅ such that each of the proportions a₁, a₂, a₃, a₄, a₅ is greater than or equal to zero and less than or equal to one and the sum a₁ + a₂ + a₃ + a₄ + a₅ is equal to one, as a solubilizing agent in aqueous solution, of a hydrophobic perfuming substance (A) or a mixture of hydrophobic perfuming substances (A), **characterized in that** said hydrophobic perfuming substance (A) is selected from the group consisting of essential oils rich in compounds having a phenolic function, said compounds having a phenolic compound being selected from the group consisting of thymol, of eugenol, isoeugenol and estragole carvacrol, and of essential oils rich in compounds having an alcohol function, said compounds having an alcohol function being selected from the group consisting of linalool, geraniol, menthol, neomenthol, cis-sabinene hydrate, citronellol, α-terpineol, viridiflorol, trans-nerolidol, α-cadinol, τ-cadinol, trans-farnesol, cis-farnesol, α-bisabolol, patchoulol and pogostol.

2. Use as defined in claim 1, **characterized in that** the weight ratio of said hydrophobic perfuming substance (A) and the composition represented by the formula (I) is greater than or equal to 1/7 and less than or equal to 2/1.

3. Aqueous solution (C₂) comprising for 100 % of its mass:
a) - from 0.05 % to 70 % by mass of at least one composition represented by the formula (I):
R-0-(G)ₚ-H (I),
wherein R represents the n-heptyl radical, G represents the residue of the sugar and p represents a decimal number greater than or equal to 1.05 and less than or equal to 2.5, said composition of formula (I) consisting of a mixture of compounds represented by formulas (I₁), (I₂), (I₃), (I₄) and (I₅):
R-O-(G)₁-H (I₁)
R-O-(G)₂-H (I₂)
R-O-(G)₃-H (I₃)
R-O-(G)₄-H (I₄)
R-O-(G)₅-H (I₅)
in the respective molar proportions a₁, a₂, a₃, a₄, a₅ such that the sum a₁ + a₂ + a₃ + a₄ + a₅ is equal to one and that each proportion a₁, a₂, a₃, a₄, a₅ is greater than or equal to zero and less than or equal to one;
**b)** - from 0.1 % to 10 % by mass of at least one of essential oil rich in compounds having a phenolic function or having an alcohol function, said compounds having a phenolic function being selected from the group consisting of thymol and of eugenol, isoeugenol and estragole carvacrol, and said compounds having an alcohol function being selected from the group consisting of linalool, geraniol, menthol, neomenthol, cis-sabinene hydrate, citronellol, α-terpineol, viridiflorol, trans-nerolidol, α-cadinol, τ-cadinol, trans-farnesol, cis-farnesol, α-bisabolol, patchoulol and pogostol, or a mixture of these oils;
**c)** - from 20 % to 99.85 % by mass of water.

4. Aqueous solution (C₂) as defined in claim 3, **characterised in that** the weight ratio of said hydrophobic perfuming substance (A) and the composition represented by the formula (I) is greater than or equal to 1/7 and less than or equal to 2/1.

5. Aqueous solution (C₂) as defined in either of claims 3 or 4, **characterised in that** its turbidity is greater than or equal to 1 NTU and less than or equal to 100 NTU, measured quantitatively by an optical nephelometry method.

6. A method for perfuming the skin, hair, scalp, mucous membranes or a garment, **characterised in that** it comprises a step of applying to said skin, said hair, said scalp, said mucous membranes or said garment the aqueous solution (C₂) as defined in any one of claims 4 to 6.

7. Use of the aqueous solution (C₂) as defined in any one of claims 4 to 6 as a perfuming agent for preparing a cosmetic composition (C₃) for topical use.
